(19) Europäisches Patentamt
European Patent Office
Office européen des brevets

(11) EP 0 673 928 B1

(12) FASCICULE DE BREVET EUROPEEN

(45) Date de publication et mention de la délivrance du brevet: **29.08.2001 Bulletin 2001/35**

(51) Int Cl.[7]: **C07D 211/52**, C07D 211/58, C07D 211/70, C07D 401/04, C07D 401/12, A61K 31/445

(21) Numéro de dépôt: **95400590.6**

(22) Date de dépôt: **17.03.1995**

(54) **Nouveaux dérivés de la N-(3,4-dichlorophényl-propyl)-pipéridine comme antagonistes sélectifs du récepteur NK3 humain**

Neue N-(3,4-Dichlorphenyl-propyl)-piperidin-Derivate als selektive humane NK3-Rezeptorantagonisten

Novel N-(3,4-dichlorophenyl-propyl)-piperidine derivatives as selective human NK3-receptor antagonists

(84) Etats contractants désignés:
**AT BE CH DE DK ES FR GB GR IE IT LI LU MC NL PT SE**
Etats d'extension désignés:
**LT SI**

(30) Priorité: **18.03.1994 FR 9403193**
**29.07.1994 FR 9409478**
**19.01.1995 FR 9500571**

(43) Date de publication de la demande:
**27.09.1995 Bulletin 1995/39**

(73) Titulaire: **SANOFI-SYNTHELABO**
**75013 Paris (FR)**

(72) Inventeurs:
- **Bichon, Daniel**
  **F-34009 Montpellier (FR)**
- **van Broeck, Didier**
  **F-34570 Murviel les Montpellier (FR)**
- **Proietto, Vincenzo**
  **F-34680 Saint Georges d'Orques (FR)**
- **Gueule, Patrick**
  **F-34820 Teyran (FR)**
- **Emonds-Alt, Xavier**
  **F-34980 Combaillaux (FR)**

(74) Mandataire: **Gillard, Marie-Louise et al**
**Cabinet Beau de Loménie**
**158, rue de l'Université**
**75340 Paris Cédex 07 (FR)**

(56) Documents cités:
**EP-A- 0 474 561** **EP-A- 0 512 901**
**EP-A- 0 515 240** **WO-A-93/18002**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention a pour objet des nouveaux composés antagonistes sélectifs du récepteur $NK_3$ humain et leur utilisation pour la préparation de médicaments utiles dans le traitement de maladies psychiatriques, de maladies d'origine psychosomatique, de l'hypertension et d'une manière générale de toute pathologie centrale ou périphérique dans laquelle la neurokinine B et le récepteur $NK_3$ interviennent dans les régulations intemeuronales.

Dans les dernières années, de nombreux travaux de recherche ont été effectués sur les tachykinines et leurs récepteurs. Les tachykinines sont distribuées à la fois dans le système nerveux central et dans le système nerveux périphérique. Les récepteurs aux tachykinines ont été reconnus et sont classés en trois types : $NK_1$, $NK_2$, $NK_3$. La substance P (SP) est le ligand endogène des récepteurs $NK_1$, la neurokinine A ($NK_A$) celui des récepteurs $NK_2$ et la neurokinine B ($NK_B$), celui des récepteurs $NK_3$.

Les récepteurs $NK_1$, $NK_2$, $NK_3$ ont été mis en évidence chez différentes espèces. Ainsi, les récepteurs $NK_3$ ont été identifiés chez le cobaye, le rat, le singe (Br. J. Pharmacol., 1990, 99, 767-773 ; Neurochem. Int., 1991, 18, 149-165) ; plus récemment, ils ont également été mis en évidence chez l'homme (FEBS Letters, 1992, 299 (1), 90-95).

Une revue récente de C.A. Maggi et al. fait le point sur les récepteurs aux tachykinines et leurs antagonistes et expose les études pharmacologiques et les applications en thérapeutique humaine (J. Autonomic Pharmacol., 1993, 13, 23-93).

Parmi les antagonistes spécifiques du récepteur $NK_1$ on peut citer les composés non peptidiques suivants : CP-96345 (J. Med. Chem., 1992, 35, 2591-2600), RP-68651 (Proc. Natl. Acad. Sci. USA, 1991, 88, 10208-10212), SR 140333 (Curr. J. Pharmacol., 1993, 250, 403-413).

Pour le récepteur $NK_2$, un antagoniste sélectif non peptidique, le SR 48968 a été décrit en détail (Life Sci., 1992, 50, PL101-PL106).

En ce qui concerne le récepteur $NK_3$, certains composés non peptidiques, antagonistes de l'Angiotensine II, ont été décrits, à ce jour, comme ayant une affinité pour le récepteur $NK_3$ du cerveau de rat et de cobaye ; cette affinité est très faible et correspond à une constante d'inhibition Ki de l'ordre de $10^{-5}$M (FASEB J., 1993, 7 (4), A 710, 4104). Un antagoniste peptidique [$Trp^7$, $Ala^8$]$NK_A$, faiblement spécifique du récepteur $NK_3$ du rat a également été décrit (J. Autonomic Pharmacol., 1993, 13, 23-93).

Dans la demande de brevet EP-A-0 512901, il est indiqué que le chlorhydrate de 5-[2-(4-hydroxy-4-phénylpipérid-1-yl)éthyl]-5-(3,4-dichlorophényl)-1-benzylpipérid-2-one, ci-après dénommé composé A antagonise la liaison de l'élédoïsine avec un Ki de 200 nanomolaire, l'élédoïsine étant un peptide d'origine batracienne équivalent à la neurokinine B. Cette demande décrit aussi la 3-[3-(4-phényl-4-acétamidopipérid-1-yl)propyl]-3-(3,4-dichlorophényl)-1-benzoylpipéridine et la 3-[3-(4-hydroxy-4-phénylpipérid-1-yl)propyl]-3-(3,4-dichlorophényl)-1-(3-méthoxybenzyl)pipéridine.

La demande de brevet EP-A-0 474 561 décrit des antagonistes des neurokinines, plus particulièrement des antagonistes des récepteurs $NK_1$ ou $NK_2$ ; notamment cette demande décrit le chlorhydrate de N-méthyl-N-[2-(3,4-dichlorophényl)-5-(4-hydroxy-4-phénylpipérid-1-yl)pentyl]benzamide.

La demande de brevet WO-A-93 18002 sécrit des sels d'ammonium quaternaire qui sont des antagonistes de la substance P. Cette demande décrit aussi, comme intermédiaire de préparation d'un de ces sels, le N-méthyl N-[5-(4-phényl-4-acétamidopipérid-1-yl)-2-(3,4-dichlorophényl)pentyl]benzyloxycarboxamide.

La demande de brevet EP-A-0 515 240 décrit des dérivés N-alkylènepipéridino monosubstitués en position 4 qui possèdent des propriétés en tant qu'antagonistes des récepteurs des neurokinines.

Les études pharmacologiques des antagonistes peptidiques et non-peptidiques des récepteurs $NK_1$ et $NK_2$ ont montré que leurs affinités pour ces récepteurs ainsi que leurs activités pharmacologiques étaient très fortement fonction de l'espèce, très probablement suite à de faibles différences dans les séquences d'acides aminés, induisant ainsi de très fines variations structurales de ces récepteurs d'une espèce à l'autre (J. Autonomic Pharmacol., 1993, 13, 23-93). Certaines données expérimentales, confirmées par la caractérisation pharmacologique des composés objets de la présente invention, semblent indiquer qu'une situation comparable existe pour le récepteur $NK_3$. En particulier, le récepteur $NK_3$ humain se distingue du récepteur $NK_3$ du rat.

On a maintenant trouvé des composés non peptidiques qui présentent une très forte affinité pour le récepteur $NK_3$ humain et une grande spécificité pour ledit récepteur. Ces composés peuvent être utilisés pour la préparation de médicaments utiles dans le traitement de maladies psychiatriques ou d'origine psychosomatique et de toutes maladies centrales ou périphériques dans lesquelles la neurokinine B et le récepteur $NK_3$ interviennent dans les régulations interneuronales.

Par très forte affinité pour le récepteur $NK_3$ humain on entend une affinité caractérisée par une constante d'inhibition Ki généralement inférieure à $5.10^{-9}$M.

Dans les études de fixation d'un ligand, la constante d'inhibition Ki est définie par la relation de Cheng-Prusoff (in Receptor Binding in Drug Research, eds. R.A. O'BRIEN. Marcel Dekker, New York, 1986) :

$$Ki = \frac{IC_{50}}{1+\frac{[L]}{Kd}}$$

[L] : concentration du ligand,
Kd : constante de dissociation du ligand,
$IC_{50}$ : concentration qui inhibe 50 % de la fixation du ligand.

Par grande spécificité pour le récepteur $NK_3$ humain, on entend que la constante d'inhibition (Ki) pour le récepteur $NK_3$ humain est généralement au moins 100 fois inférieure à la constante d'inhibition (Ki) pour le récepteur $NK_2$ ou à celle pour le récepteur $NK_1$ de différentes espèces.

Par maladie d'origine psychosomatique, on désigne, des maladies ayant leur origine dans le système nerveux central (SNC) et des conséquences pathologiques au niveau périphérique.

Ainsi selon un de ses aspects, la présente invention a pour objet les composés suivants :

la 1-benzoyl-3-(3,4-dichlorophényl)-3-[3-(4-(acétyl-N-méthylamino)-4-phénylpipéridin-1-yl)-propyl]pipéridine,
la 1-benzoyl-3-(3,4-dichlorophényl)-3-[3-(4-propionylamino-4-phénylpipéridin-1-yl)-propyl]pipéridine,
la 1-benzoyl-3-(3,4-dichlorophényl)-3-[3-(4-(propionyl-N-méthylamino)-4-phénylpipéridin-1-yl)-propyl]pipéridine,
la 1-benzoyl-3-(3,4-dichlorophényl)-3-[3-(4-butyrylamino-4-phénylpipéridin-1-yl)-propyl]pipéridine,
la 1-benzoyl-3-(3,4-dichlorophényl)-3-[3-(4-(butyryl-N-méthylamino)-4-phénylpipéridin-1-yl)-propyl]pipéridine,
la 1-benzoyl-3-(3,4-dichlorophényl)-3-[3-(4-isobutyrylamino-4-phénylpipéridin-1-yl)-propyl]pipéridine,
la 1-benzoyl-3-(3,4-dichlorophényl)-3-[3-(4-(isobutyryl-N-méthylamino)-4-phénylpipéridin-1-yl)-propyl]pipéridine,
la 1-benzoyl-3-(3,4-dichlorophényl)-3-[3-(4-valérylamino-4-phénylpipéridin-1-yl)-propyl]pipéridine,
la 1-benzoyl-3-(3,4-dichlorophényl)-3-[3-(4-(valéryl-N-méthylamino)-4-phénylpipéridin-1-yl)-propyl]pipéridine,
la 1-benzoyl-3-(3,4-dichlorophényl)-3-[3-(4-isovalérylamino-4-phénylpipéridin-1-yl)-propyl]pipéridine,
la 1-benzoyl-3-(3,4-dichlorophényl)-3-[3-(4-(isovaléryl-N-méthylamino)-4-phénylpipéridin-1-yl)-propyl]pipéridine,
la 1-benzoyl-3-(3,4-dichlorophényl)-3-[3-(4-pivaloylamino-4-phényl-pipéridin-1-yl)-propyl]pipéridine,
la 1-benzoyl-3-(3,4-dichlorophényl)-3-[3-(4-(pivaloyl-N-méthylamino)-4-phénylpipéridin-1-yl)-propyl]pipéridine,

sous forme de racémates, d'énantiomères (+) ou (-) ou de sels.

Le chlorhydrate de la 1-benzoyl-3-(3,4-dichlorophényl)-3-[3-(4-(acétyl-N-méthylamino)-4-phénylpipéridin-1-yl)-propyl] pipéridine, sous forme optiquement pure, préférentiellement sous forme de l'isomère(+), est tout particulièrement préféré.

Les sels mentionnés ci-dessus comprennent aussi bien ceux avec des acides minéraux ou organiques qui permettent une séparation ou une cristallisation convenables des composés de l'invention, tels que l'acide picrique ou l'acide oxalique ou un acide optiquement actif, par exemple, l'acide mandélique ou camphosulfonique que ceux qui forment des sels pharmaceutiquement acceptables.

Les sels pharmaceutiquement acceptables sont tels que le chlorhydrate, le bromhydrate, le sulfate, l'hydrogénosulfate, le dihydrogénophosphate, le méthanesulfonate, le méthylsulfate, le maléate, le fumarate, le 2-naphtalènesulfonate, le benzènesulfonate, le glycolate, le gluconate, le citrate, l'iséthionate, le *para*toluènesulfonate.

La présente invention englobe les composés ci-dessus soit sous forme racémique, soit sous forme énantiomérique pure.

Les composés selon l'invention sont obtenus par des méthodes connues en particulier celles qui sont décrites dans les demandes de brevet EP-A-0 474 561 et EP-A-0 512 901.

Un des procédés qui convient pour l'obtention des composés de l'invention est décrit ci-après. Selon ce procédé

a) on traite un composé de formule :

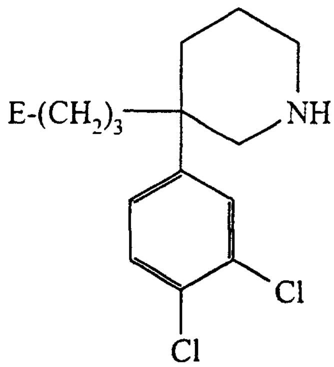

1

dans laquelle E représente un hydroxyle ou éventuellement un groupe O-protégé tel que par exemple un tétrahydropyran-2-yloxy, un benzoyloxy ou un ($C_1$-$C_4$)alkylcarbonyloxy ou un groupe :

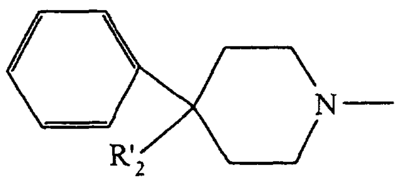

2

dans lequel $R'_2$ est tel que défini dans la revendication 3, avec un dérivé fonctionnel d'un acide de formule :

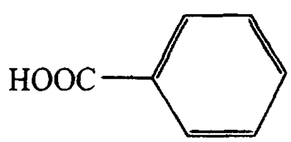

3

pour obtenir un composé de formule :

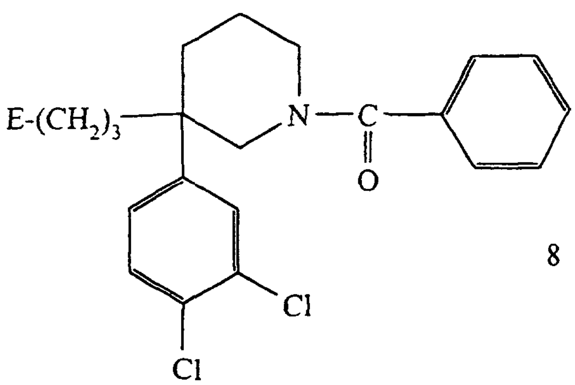

8

b) on élimine éventuellement le groupe O-protecteur par action d'un acide ou d'une base,

c) on traite l'alcool ainsi obtenu de formule :

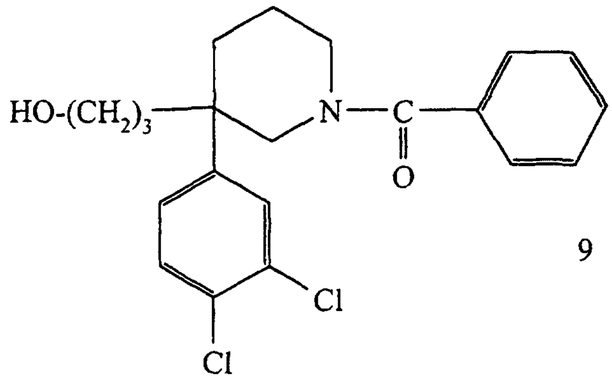

9

avec un composé de formule:

$W\text{-}SO_2\text{-}Cl$ 9a

dans laquelle W représente un groupe méthyle, phényle, tolyle, trifluorométhyle,
d) on fait réagir le sulfonate ainsi obtenu de formule :

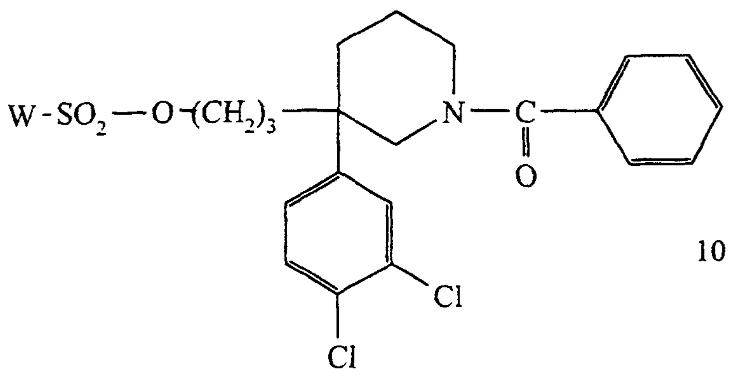

10

avec une amine secondaire de formule :

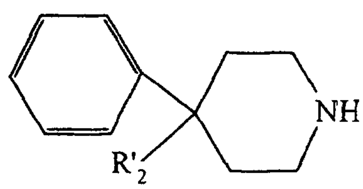

11

dans laquelle $R'_2$ est tel que défini ci-dessus,
e) après déprotection éventuelle de l'hydroxyle ou de l'amino représenté par $R'_2$, on transforme éventuellement le produit obtenu en l'un de ses sels.

Dans l'étape c) le composé 9a est avantageusement le chlorure de méthanesulfonyle et donc, dans le composé 10 de l'étape d), W est avantageusement un groupe méthyle.
Lorsque dans le composé de formule 1, E représente un groupe :

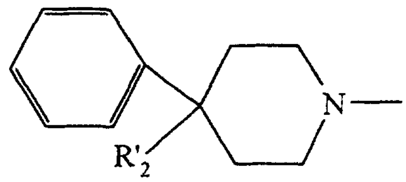

le procédé comprend uniquement les étapes a) et e).

Selon une variante du procédé,

a1) on protège la fonction amine du composé de formule 1 par un groupe protecteur de façon à préparer un composé de formule :

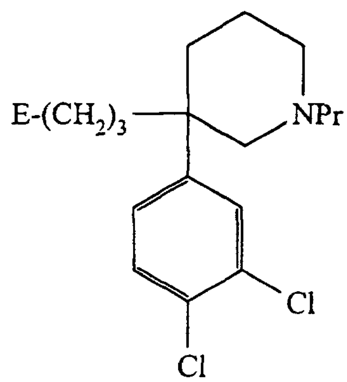

12

dans laquelle E est tel que défini précédemment et Pr représente un groupe protecteur de l'azote par exemple un *tert*-butoxycarbonyle (Boc), un trityle, un benzyle,

b1) on élimine éventuellement le groupe O-protecteur par action d'un acide ou d'une base,

c1) on traite l'alcool ainsi obtenu de formule :

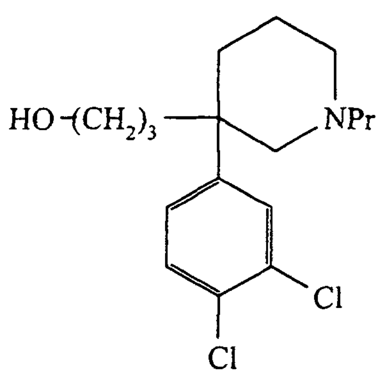

13

avec un composé de formule 9a décrit ci-dessus,

d1) on fait réagir le sulfonate ainsi obtenu de formule :

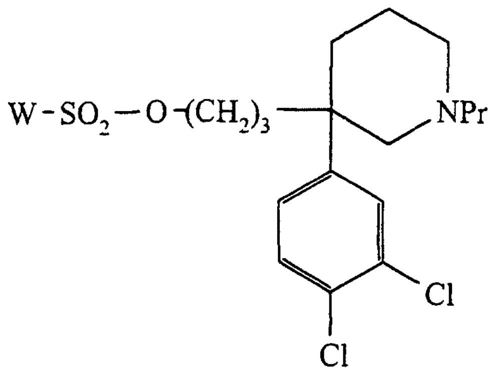

14

avec une amine secondaire :

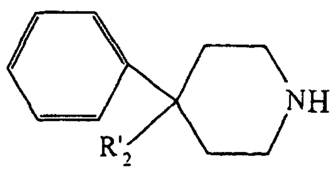

11

pour obtenir le composé de formule :

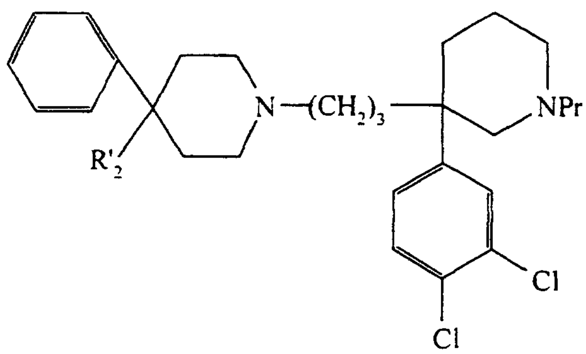

15

e1) on effectue la déprotection de l'azote en milieu acide,
f1) on traite le composé ainsi obtenu de formule :

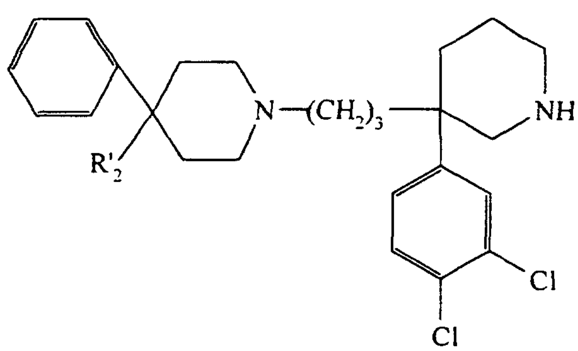

16

par le composé 3 décrit ci-dessus,
g1) après déprotection éventuelle de l'hydroxyle ou de l'amino représenté par $R'_2$, on transforme éventuellement le produit obtenu en l'un de ses sels.

Dans l'étape c1) le composé 9a est avantageusement le chlorure de méthanesulfonyle et donc, dans le composé 14 de l'étape d1), W est avantageusement un groupe méthyle.

Des conditions opératoires particulièrement avantageuses des étapes ci-dessus sont explicitées ci-après et illustrées dans les exemples.

Dans l'étape a), le dérivé fonctionnel de l'acide 3, peut être l'acide lui-même, ou bien un des dérivés fonctionnels qui réagissent normalement avec les amines, par exemple un anhydride, un anhydride mixte, le chlorure d'acide, ou un ester activé, comme l'ester de paranitrophényle.

Lorsqu'on met en oeuvre l'acide de formule 3 lui-même, on opère en présence d'un agent de couplage utilisé en chimie peptidique tel que le 1,3-dicyclohexylcarbodiimide ou l'hexafluorophosphate de benzotriazol-1-yloxytris (diméthylamino)phosphonium en présence d'une base telle que la triéthylamine ou la N,N-diisopropyléthylamine, dans un solvant inerte tel que le dichlorométhane ou le N,N-diméthylformamide à une température comprise entre 0°C et la température ambiante.

Lorsqu'on utilise un chlorure d'acide, la réaction s'effectue dans un solvant inerte tel que le dichlorométhane ou le benzène, en présence d'une base telle que la triéthylamine ou la N-méthylmorpholine et à une température comprise entre -60°C et la température ambiante.

Le composé de formule 8 ainsi obtenu est éventuellement déprotégé à l'étape b) selon les méthodes connues de l'homme de l'art. Par exemple, lorsque E représente un groupe tétrahydropyran-2-yloxy, la déprotection s'effectue par hydrolyse acide en utilisant l'acide chlorhydrique dans un solvant tel que l'éther, le méthanol ou le mélange de ces solvants, ou en utilisant le *p*-toluènesulfonate de pyridinium dans un solvant tel que le méthanol ou encore, en utilisant une résine Amberlyst® dans un solvant tel que le méthanol. La réaction s'effectue à une température comprise entre la température ambiante et la température de reflux du solvant. Lorsque E représente un groupe benzoyloxy ou un groupe ($C_1$-$C_4$)alkylcarbonyloxy, la déprotection s'effectue par hydrolyse en milieu alcalin en utilisant par exemple un hydroxyde de métal alcalin tel que l'hydroxyde de sodium, l'hydroxyde de potassium ou l'hydroxyde de lithium, dans un solvant inerte tel que l'eau, le méthanol, le dioxane ou un mélange de ces solvants, à une température comprise entre 0°C et la température de reflux du solvant.

A l'étape c) la réaction de l'alcool de formule 9 avec un chlorure de sulfonyle de formule 9a s'effectue de préférence en présence d'une base telle que la triéthylamine, la pyridine, la N,N-diisopropyléthylamine ou la N-méthylmorpholine, dans un solvant inerte tel que le dichlorométhane, le benzène ou le toluène, à une température comprise entre -20°C et la température de reflux du solvant.

Lorsqu'on fait réagir un composé de formule 10 avec un composé de formule 11 (étape d), la réaction s'effectue de préférence dans un solvant inerte tel que le N,N-diméthylformamide, l'acétonitrile, le chlorure de méthylène, le toluène ou l'isopropanol et en présence ou en l'absence d'une base. Lorsqu'on utilise une base, celle-ci est choisie parmi les bases organiques telles que la triéthylamine, la N,N-diisopropyléthylamine ou la N-méthylmorpholine ou parmi les carbonates ou bicarbonates de métal alcalin tels que le carbonate de potassium, le carbonate de sodium ou le bicarbonate de sodium. En l'absence de base, la réaction s'effectue en utilisant un excès du composé de formule 11 et éventuellement en présence d'un iodure de métal alcalin tel que l'iodure de potassium ou l'iodure de sodium. La réaction s'effectue à une température comprise entre la température ambiante et 100°C.

Les produits de l'invention ainsi obtenus sont isolés sous forme de base libre ou de sel, selon les techniques classiques.

Lorsque le composé de l'invention est obtenu sous forme de base libre, la salification est effectuée par traitement avec l'acide choisi dans un solvant organique. Par traitement de la base libre, dissoute par exemple dans un éther tel que l'éther diéthylique ou dans un alcool tel que le propan-2-ol ou dans l'acétone, avec une solution de l'acide choisi dans le même solvant, on obtient le sel correspondant qui est isolé selon les techniques classiques.

Ainsi, on prépare par exemple le chlorhydrate, le bromhydrate, le sulfate, l'hydrogénosulfate, le dihydrogénophosphate, le méthanesulfonate, l'oxalate, le maléate, le fumarate, le naphtalène-2-sulfonate, le benzènesulfonate.

A la fin de la réaction, les composés de l'invention peuvent être isolés sous forme d'un de leurs sels, par exemple le chlorhydrate, ou l'oxalate ; dans ce cas, s'il est nécessaire, la base libre peut être préparée par neutralisation dudit sel avec une base minérale ou organique, telle que l'hydroxyde de sodium ou la triéthylamine ou avec un carbonate ou bicarbonate alcalin, tel que le carbonate ou bicarbonate de sodium ou de potassium.

Les pipéridines substituées de formule 11 sont connues ou préparées par des méthodes connues.

Les composés de formule 11 sont généralement préparés sous forme protégée sur l'azote de la pipéridine; ils permettent d'obtenir par une étape de déprotection les composés de formule 11 eux-mêmes.

Les composés de formule 11 dans laquelle $R'_2$ représente un groupe acylamino éventuellement substitué sur l'azote par un méthyle, s'obtiennent par action de l'acide formique dans l'anhydride acétique ou respectivement de l'anhydride approprié ou d'un chlorure d'acide approprié en présence d'une base telle que la triéthylamine, sur un composé de formule 11 dans laquelle $R'_2$ représente un groupe amino éventuellement substitué sur l'azote par un méthyle.

De façon particulière, on peut préparer un composé de formule 11 dans laquelle $R'_2$ représente un groupe

acétyl-N-méthylamino par hydrogénation, en présence d'un catalyseur tel que le palladium sur charbon, d'un composé de formule 11 dans laquelle $R'_2$ représente un groupe acryloyl-N-méthylamino.

Les méthodes ci-dessus sont bien connues et illustrées par les Préparations ci-dessous qui précèdent les EXEMPLES. Ces préparations constituent des adaptations des méhodes décrites dans EP-A-0428434, EP-A-0474561, EP-A-0512901 ou dans les publications suivantes :

J. Heterocyclic. Chem., 1986, 23, 73-75
J. Chem. Soc., 1950, 1469
J. Chem. Soc., 1945, 917
J. Pharmaceutical. Sci., 1972, 61, 1316-1317
J. Org. Chem. 1957, 22, 1484-1489.

Les composés de formule 1 dans lesquels E représente un hydroxyle sont préparés par des méthodes connues. Le SCHEMA 1 suivant résume l'une de ces méthodes pour les composés de formule 1'.

SCHEMA 1

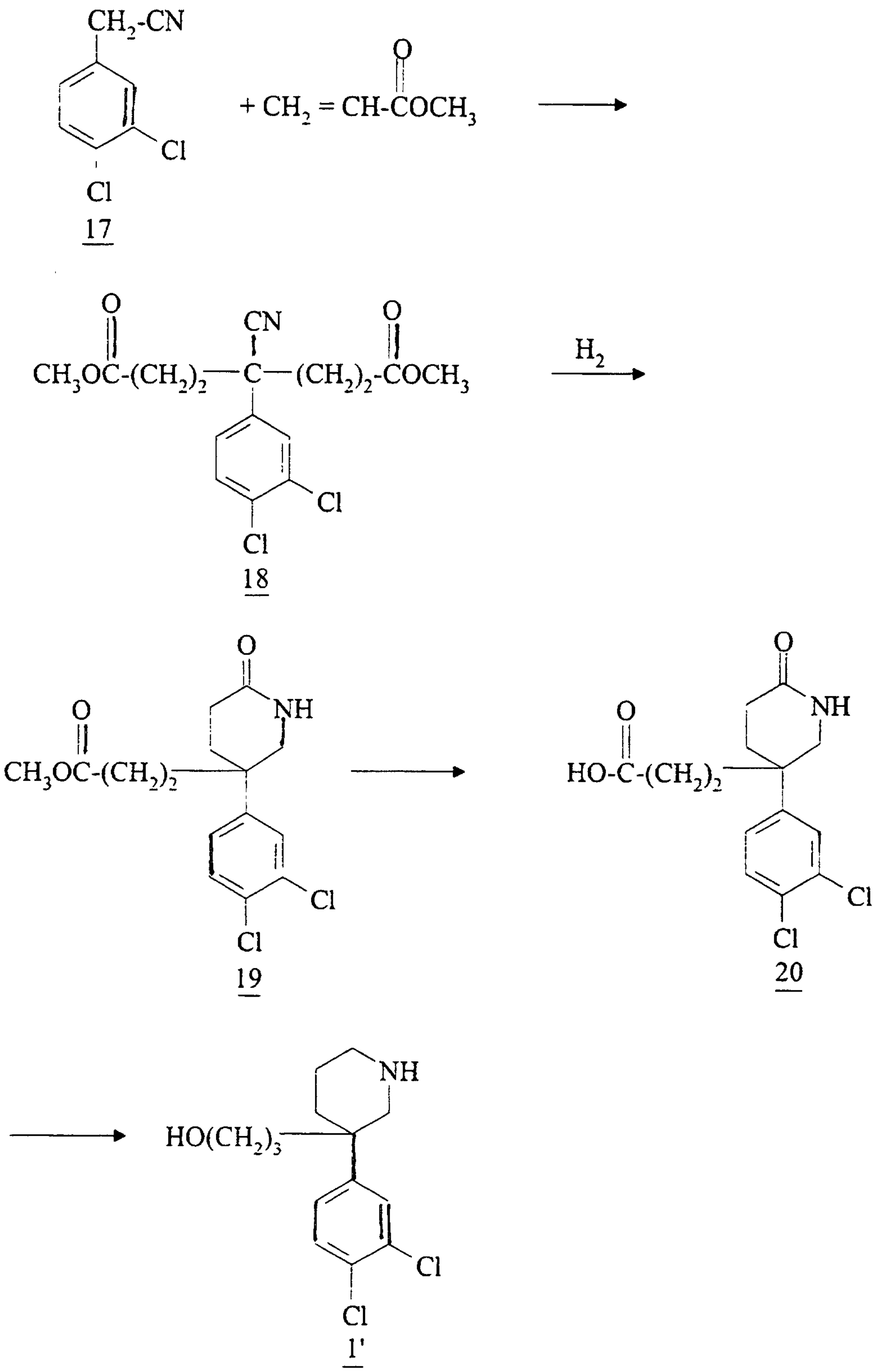


17

18

19

20

1'

Le composé 3 est connu.

Ce composé peut être utilisé sous forme marquée, par exemple au tritium, ou à l'iode radioactif, pour préparer des composés selon l'invention marqués.

Dans ce cas on opère à partir d'un composé 3 dans lequel le radical phényle est substitué par un atome

d'iode puis on échange cet atome d'iode par un tritium ou un atome d'iode radioactif pour préparer un composé marqué 3* permettant de préparer un composé de l'invention marqué.

L'alcool (1') obtenu selon le schéma 1 est racémique. On peut éventuellement effectuer la séparation de ses isomères optiques par des méthodes connues, par exemple par chromatographie ou recristallisation puis préparer le mésylate optiquement pur correspondant et préparer ainsi les composés selon l'invention sous forme optiquement pure.

L'alcool (1'), qui constitue l'intermédiaire clé dans la synthèse des composés de l'invention qui sont des antagonistes puissants et sélectifs du récepteur $NK_3$ humain, est un composé nouveau. La forme racémique de cet alcool, les deux énantiomères (+) et (-) et les sels de ces composés sont donc un autre aspect de la présente invention, l'isomère (+) et ses sels d'addition acides étant particulièrement préférés.

Selon un autre aspect, la présente invention a pour objet l'utilisation d'un composé tel que défini précédemment, pour la préparation de médicaments utiles dans le traitement de toute pathologie dans laquelle la neurokinine B intervient, en particulier pour la préparation de médicaments destinés à combattre les maladies psychiatriques, les maladies d'origine psychosomatique, l'hypertension, les pathologies liées à des désordres de neuromodulation ou neurotransmission $NK_3$ dépendante, avantageusement pour traiter l'hypertension.

L'affinité des composés de l'invention pour les récepteurs aux tachykinines a été évaluée in vitro par plusieurs essais biochimiques utilisant des radioligands :

1°) La liaison de [$^{125}$I] BH-SP (Substance P marquée à l'iode 125 à l'aide du réactif de Bolton-Hunter) aux récepteurs $NK_1$ du cortex de rat, de l'iléon de cobaye et des cellules lymphoblastiques humaines.
2°) La liaison [$^{125}$I] His-$NK_A$ aux récepteurs $NK_2$ du duodénum de rat ou de l'iléon de cobaye.
3°) La liaison [$^{125}$I] His [MePhe$^7$] $NK_B$ aux récepteurs $NK_3$ du cortex cérébral de rat, du cortex cérébral de cobaye et du cortex cérébral de gerbille ainsi qu'aux récepteurs clonés $NK_3$ humains exprimés par des cellules CHO (Buell et al., FEBS Letters, 1992, 299, 90-95).

Les essais ont été effectués selon X. Emonds-Alt et al. (Eur. J. Pharmacol,. 1993, 250, 403-413).

Les composés selon l'invention inhibent fortement la liaison de [$^{125}$I]His[MePHe$^7$] $NK_B$ aux récepteurs $NK_3$ du cortex cérébral de cobaye et de gerbille ainsi qu'aux récepteurs clonés $NK_3$ humain : la constante d'inhibition Ki est généralement inférieure à $5.10^{-9}$M. Pour les mêmes composés on a constaté que la constante d'inhibition (Ki) pour les récepteurs $NK_3$ du cortex cérébral de rat est supérieure à $10^{-7}$M et que la constante d'inhibition (Ki) pour le récepteur $NK_2$ du duodénum de rat et les récepteurs $NK_1$ du cortex de rat est généralement supérieure ou égale à $10^{-7}$ M.

A titre de comparaison, on a mesuré, selon les conditions opératoires décrites ci-dessus les constantes d'inhibition vis-à-vis des différents récepteurs pour le composé A. L'antagonisme de la liaison à l'élédoïsine décrit dans la demande EP 512901 correspond à la constante d'inhibition sur le récepteur $NK_3$ de rat :

$$Ki = 10^{-7}\ M$$

Pour le récepteur $NK_3$ humain, la constante d'inhibition du composé A est $Ki = 1.10^{-9}$ M.

Pour le récepteur $NK_2$ de duodénum de rat, la constante d'inhibition du composé A est $Ki = 1.10^{-10}$ M.

Ainsi le composé A n'est pas sélectif du récepteur $NK_3$ humain contrairement à ce qui est observé pour les composés selon la présente invention.

Le chlorhydrate de N-méthyl-N-[2-(3,4-dichlorophényl)-5-(4-hydroxy-4-phényl-pipéridin-1-yl)pentyl]benzamide décrit à l'exemple 22 de la demande EP 474561 présente des constantes d'inhibition montrant la grande spécificité et la forte affinité de ce composé pour le récepteur $NK_3$ humain :

récepteur $NK_3$ humain, $Ki = 5.10^{-9}$ M
récepteur $NK_2$ de duodénum de rat, $Ki = 5.10^{-7}$ M
récepteur $NK_1$ de cortex de rat, $Ki = 5.10^{-7}$ M.

Les composés selon la présente invention ont également été évalués in vivo sur deux modèles animaux.

Chez le gerbille, un comportement de rotation est induit par administration intrastriatale d'un agoniste spécifique du récepteur $NK_3$ : le senktide ; on a constaté qu'une application unilatérale de senktide dans le striatum de gerbille conduit à de fortes rotations contralatérales qui sont inhibées par les composés selon l'invention administrés soit par voie intrapéritonéale, soit par voie orale.

Ce résultat montre que les composés selon l'invention passent la barrière hématoméningée et qu'ils sont susceptibles de bloquer, au niveau du système nerveux central, l'action propre aux récepteurs $NK_3$. Ils pourront ainsi être utilisés pour le traitement de toute pathologie centrale $NK_B$ dépendante, tel que les maladies psychiatriques, ou

de toute pathologie médiée au niveau central par le récepteur $NK_3$, telle que les maladies psychosomatiques.

Chez le cobaye, une injection de senktide par voie intraveineuse ou intracérébroventriculaire induit une hypertension qui est supprimée par l'administration par voie orale ou intraveineuse des composés selon l'invention.

Ce résultat montre que les composés selon l'invention agissent au niveau cardiovasculaire et qu'ils sont capables de bloquer l'action propre aux récepteurs $NK_3$ à ce niveau, notamment l'hypertension (Nakayama et al., Brain Res. 1992, 595 339-342, Takano and Kamiya, Asia Pacific. J. Pharmacol., 1991, 6, 341-346, Saigo et al., Neuroscience Letters, 1993, 159, 187-190).

Dans ces tests, les composés selon l'invention sont actifs à des doses variant de 0,1 mg à 3 mg par kg par voie orale, intraveineuse ou intrapéritonéale.

Les composés utiles pour la préparation de médicaments selon l'invention sont généralement administrés en unité de dosage. Lesdites unités de dosage sont de préférence formulées dans des compositions pharmaceutiques dans lesquelles le principe actif est mélangé avec un excipient pharmaceutique.

Selon un autre de ses aspects, la présente invention concerne des compositions pharmaceutiques renfermant, en tant que principe actif, un composé de l'invention, ayant une affinité très forte pour le récepteur $NK_3$ humain, caractérisée par une constante d'inhibition Ki inférieure à $5.10^{-9}$M dans les études de fixation du ligand.

Les composés de l'invention et leurs sels pharmaceutiquement acceptables peuvent être utilisés à des doses journalières de 0,01 à 100 mg par kilo de poids corporel du mammifère à traiter, préférentiellement à des doses journalières de 0,1 à 50 mg/kg. Chez l'être humain, la dose peut varier de préférence de 0,5 à 4000 mg par jour, plus particulièrement de 2,5 à 1000 mg selon l'âge du sujet à traiter ou le type de traitement : prophylactique ou curatif.

Les maladies pour le traitement desquelles les composés de l'invention et leurs sels pharmaceutiquement acceptables peuvent être utilisés, sont par exemple les maladies associés à un dysfonctionnement des systèmes dopaminergiques telle que la schizophrénie, la maladie de Parkinson, les maladies associés à un dysfonctionnement des systèmes noradrénergiques tels que l'anxiété, les troubles de la vigilance ainsi que les maladies épileptiques de toute forme et en particulier le Grand Mal, la démence, les maladies neurodégénératives, et les maladies périphériques dans lesquelles la participation du système nerveux central et/ou du système nerveux périphérique se fait par l'intermédiaire de la neurokinine B agissant comme neurotransmetteur ou neuromodulateur central tels que la douleur, la migraine, l'inflammation aigue ou chronique, les troubles cardiovasculaires en particulier l'hypertension, l'insuffisance cardiaque, et les troubles du rythme, les troubles respiratoires (asthme, rhinite, toux, bronchites, allergies, hypersensibilité), les troubles du système gastrointestinal tels que ulcère oesophagique, colite, désordres liés au stress (stress-related disorders), syndrome du colon irritable (IBS), les maladies inflammatoires du colon (IBD), hypersécrétion acide (acidic secretion), les troubles du système urinaire (incontinence, vessie neurologique), les maladies du système immunitaire (arthrite rhumatoide), et plus généralement toute pathologie neurokinine B dépendante.

Dans les compositions pharmaceutiques de la présente invention pour l'administration par voie orale, sublinguale, inhalée, sous-cutanée, intramusculaire, intraveineuse, transdermique, locale ou rectale, les principes actifs peuvent être administrés sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange le principe actif principal avec un véhicule pharmaceutique tel que la silice, la gélatine, l'amidon, le lactose, le stéarate de magnésium, le talc, la gomme arabique ou analogues. On peut enrober les comprimés de saccharose de divers polymères ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant tel qu'un glycol ou un ester de glycerol et en incorporant le mélange obtenu dans des gélules molles ou dures.

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec de liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions injectables qui contiennent des agents de dispersion et/ou des agents mouillants pharmacologiquement compatibles, par exemple le propylèneglycol ou le butylèneglycol.

Pour une administration par inhalation on utilise un aérosol contenant en outre, par exemple du trioléate de

sorbitane ou de l'acide oléique ainsi que du trichlorofluorométhane, du dichlorofluorométhane, du dichlorotétrafluoroéthane ou tout autre gaz propulseur biologiquement compatible ; on peut également utiliser un système contenant le principe actif, seul ou associé à un excipient, sous forme de poudre.

Le principe actif peut être également présenté sous forme de complexe avec une cyclodextrine, par exemple, α-, β-, γ-cyclodextrine, 2-hydroxypropyl-β-cyclodextrine, méthyl-β-cyclodextrine.

Le principe actif peut être formulé également sous forme de microcapsules, éventuellement avec un ou plusieurs supports ou additifs.

Dans chaque unité de dosage le principe actif est présent dans les quantités adaptées aux doses journalières envisagées. En général chaque unité de dosage est convenablement ajustée selon le dosage et le type d'administration prévu, par exemple comprimés, gélules et similaires, sachets, ampoules, sirops et similaires, gouttes de façon à ce qu'une telle unité de dosage contienne de 0,5 à 1000 mg de principe actif, de préférence de 2,5 à 250 mg devant être administrés une à quatre fois par jour.

Les compositions susdites peuvent également renfermer d'autres produits actifs utiles pour la thérapeutique souhaitée tels que, par exemple, des bronchodilatateurs, des antitussifs ou des antihistaminiques.

Grâce à leur très forte affinité pour le récepteur $NK_3$ humain et à leur grande sélectivité, les composés selon l'invention pourront être utilisés, sous forme radiomarquée comme réactifs de laboratoire.

Par exemple, ils permettent d'effectuer la caractérisation, l'identification et la localisation du récepteur $NK_3$ humain dans des coupes de tissus, ou du récepteur $NK_3$ chez l'animal entier par autoradiographie.

Les composés selon l'invention permettent également d'effectuer le tri ou screening des molécules en fonction de leur affinité pour le récepteur $NK_3$ humain. On opère alors par une réaction de déplacement du ligand radiomarqué, objet de la présente invention de son récepteur $NK_3$ humain.

Dans les exemples qui vont suivre, les abréviations suivantes sont utilisées :

TA : température ambiante
F : point de fusion
TEA : triéthylamine
Pd/C : Palladium sur charbon à 10 %
DCM : dichlorométhane
THF : tétrahydrofurane
DBU : 1,8-diazabicyclo[5.4.0]undec-7-ène
DMAP : 4-diméthylaminopyridine
AcOEt : acétate d'éthyle
MeOH : méthanol
HPLC : chromatographie liquide sous haute pression
iPr : isopropyle
Bu : n-butyle
$C_6H_5$ : phényle
HCl : acide chlorhydrique
$(Boc)_2O$ : di-*tert*-butyldicarbonate
Boc = *tert*-butoxycarbonyl
NaCl : chlorure de sodium
$MgSO_4$ : sulfate de magnésium
$LiAlH_4$ : hydrure d'aluminium et de lithium
EtOH : éthanol
$Na_2CO_3$: carbonate de sodium
NaOH : soude
éther : éther diéthylique
$K_2CO_3$ : carbonate de potassium
éther chlorhydrique : solution saturée d'HCl dans l'éther.
silice H : gel de silice 60 H commercialisé par Merck (DARMSTAD).

PREPARATIONS

PREPARATION 1.1 :

4-Phényl-4-pivaloylaminopipéridine.

A) 1-Benzyl-4-hydroxy-4-phénylpipéridine

Ce composé est préparé par action du phényllithium sur la 1-benzylpipéridin-4-one.

B) 4-Acétamido-1-benzyl-4-phénylpipéridine.

Ce composé est obtenu selon la réaction de Ritter par addition d'acétonitrile sur le composé préparé à l'étape A.

C) Dichlorhydrate de 4-amino-1-benzyl-4-phénylpipéridine.

On hydrolyse à reflux pendant 3 heures dans HCl 6N, le composé préparé à l'étape B. Après évaporation à sec, on dissout le résidu dans le méthanol, cristallise par addition d'acétone, filtre et sèche pour obtenir le composé attendu.

D) 1-Benzyl-4-phényl-4-pivaloylaminopipéridine.

On dissout 70 g du composé préparé à l'étape précédente dans 150 ml de dioxane, ajoute 85 ml de TEA, puis 45 g de chlorure de pivaloyle. Après 2 heures sous agitation à 60°C, on évapore, reprend dans DCM, lave par de la soude diluée, par une solution de NaCl puis sèche sur $MgSO_4$ et évapore. Le résidu est chromatographié sur silice en éluant par DCM. On obtient 43 g du produit attendu sous forme d'huile.

E) 4-Phényl-4-pivaloylaminopipéridine.

13 g du composé obtenu à l'étape précédente sont dissous dans 20 ml d'éthanol à 95 % ; on ajoute 1,5 g de Pd/C puis on hydrogène pendant 24 heures à température ambiante, sous pression atmosphérique, on filtre, évapore, pour obtenir une huile qui cristallise en donnant 8 g de produit attendu.

PREPARATION 1.2:

4-Phényl-4-(pivaloyl-N-méthylamino)pipéridine.

A) 1-Benzyl-4-(N-*tert*-butoxycarbonylamino)-4-phénylpipéridine.

A une solution de 14,5 g de dichlorhydrate de 4-amino-1-benzyl-4-phénylpipéridine obtenu à l'étape C de la Préparation 1,1, 12 ml de TEA dans 100 ml de dioxane, on ajoute, goutte à goutte, une solution de 12 g de $(Boc)_2O$ dans 50 ml de dioxane et chauffe pendant 18 heures à 50°C. On concentre sous vide le mélange réactionnel, extrait le résidu à l'AcOEt, lave par une solution tampon pH = 2, par une solution de NaOH 1N, sèche sur $MgSO_4$ et évapore sous vide le solvant. On obtient 13 g du produit attendu après cristallisation dans le mélange éther/heptane.

B) 4-(N-*tert*-butoxycarbonylamino)-4-phénylpipéridine.

On hydrogène pendant 72 heures à TA et à pression atmosphérique un mélange de 13 g du composé obtenu à l'étape précédente, 1,5 g de palladium sur charbon à 10 % dans 300 ml d'EtOH 95. On filtre le catalyseur sur Célite et évapore sous vide le filtrat. On obtient 9,7 g du produit attendu.

C) 4-(N-*tert*-butoxycarbonylamino)-4-phényl-1-tritylpipéridine.

13,25 g du composé obtenu à l'étape précédente et 5 g de TEA sont dissous dans 150 ml de DCM à 0°C, sous azote. On ajoute goutte à goutte 13,4 g de chlorure de trityle et laisse 1 heure sous agitation. On évapore, reprend par de l'éther, lave à l'eau, par une solution tampon à pH 2, une solution de NaCl, puis sèche sur $MgSO_4$ et évapore. On obtient 23 g du produit attendu sous forme d'huile.

D) 4-(N-Méthylamino)-4-phényl-1-tritylpipéridine.

On chauffe à 60°C, sous azote, une suspension de 5 g de $LiAlH_4$ dans 100 ml de THF et ajoute, goutte à goutte, une solution de 23 g du composé obtenu à l'étape précédente dans 100 ml de THF. Après 2 heures au reflux, on hydrolyse par 25 ml d'eau, filtre, évapore. On obtient 17 g du produit attendu qui cristallise dans du méthanol à chaud, F = 125°C.

E) 4-(Pivaloyl-N-méthylamino)-4-phényl-1-tritylpipéridine.

2,6 g du composé obtenu à l'étape précédente sont dissous dans 15 ml de pyridine, on ajoute 500 mg de DMAP et 4 ml de chlorure de pivaloyle. On laisse réagir 72 heures à 70°C sous azote puis on évapore, dissout dans AcOEt, lave par de l'eau, une solution tampon à pH = 2, une solution de soude à 5 %, une solution de NaCl, puis on sèche sur $MgSO_4$. Le résidu est chromatographié sur silice en éluant par un mélange pentane/AcOEt. On obtient le produit attendu (1,5 g) sous forme d'huile.

F) 4-Phényl-4-(Pivaloyl-N-méthylamino)pipéridine.

1,5 g du produit obtenu à l'étape précédente sont dissous dans un mélange de 20 ml d'acide formique et 20 ml d'eau. Après 1 heure sous agitation, on filtre, neutralise le filtrat par addition à froid d'une solution de NaOH à 40 % puis extrait 3 fois par 50 ml de DCM ; on sèche sur $MgSO_4$ et évapore. On obtient 700 mg du produit attendu sous forme d'un produit pâteux, F = 50-55°C.

PREPARATION 1.3

4-(Acétyl-N-méthylamino)-4-phénylpipéridine.

A) 4-(Acétyl-N-méthylamino)-4-phényl-1-tritylpipéridine.

On refroidit à 0°C, sous azote, une solution de 2,8 g du composé obtenu à l'étape D de la Préparation 1.2 dans 20 ml de DCM et ajoute 1,5 ml de TEA puis 0,55 g de chlorure d'acétyle. On laisse 2 heures sous agitation et concentre sous vide le mélange réactionnel. On reprend le résidu à l'AcOEt, lave par une solution tampon pH = 2, par une solution de NaOH à 5 %, par une solution saturée de NaCl, sèche sur $MgSO_4$ et évapore sous vide le solvant. On obtient 3 g du produit attendu.

B) 4-(Acétyl-N-méthylamino)-4-phénylpipéridine.

On chauffe à 60°C pendant 1 heure une solution de 3 g du composé obtenu à l'étape précédente, 30 ml d'acide formique dans 15 ml d'eau. On ajoute 50 ml d'eau au mélange réactionnel, filtre, lave le filtrat à l'éther, alcanilise la phase aqueuse à pH > 10 par ajout d'une solution de NaOH concentrée, extrait au DCM, sèche sur $MgSO_4$ et évapore sous vide le solvant. On obtient 1 g du produit attendu sous forme d'huile.

PREPARATION 1.4

p-Toluènesulfonate de 4-(cyclopropylcarbonyl-N-méthylamino)-4-phényl pipéridine.

A) 1-Benzyl-4-(N-méthylamino)-4-phénylpipéridine.

A une suspension de 12,5 g d'hydrure d'aluminium et de lithium dans 100 ml de THF, on ajoute goutte à goutte, une solution de 38,8 g du composé obtenu à l'étape A de la PREPARATION 1.8 (sous forme de base libre, F = 140°C) dans 400 ml de THF et on chauffe à reflux pendant 3 heures. On hydrolyse par ajout d'une solution de 5 ml de NaOH concentrée dans 45 ml d'eau, filtre les sels minéraux et concentre sous vide le filtrat. On obtient 38 g du produit attendu que l'on utilise tel quel.

B) 1-Benzyl-4-(cyclopropylcarbonyl-N-méthylamino)-4-phénylpipéridine.

On refroidit à 0°C une solution de 1,5 g du composé obtenu à l'étape précédente, 1,5 ml de triéthylamine dans 20 ml de DCM, ajoute, goutte à goutte, 0,58 ml de chlorure de cyclopropanecarbonyle et laisse 2 heures sous agitation en laissant remonter la température à TA. On lave le mélange réactionnel deux fois à l'eau, par une solution de NaOH 1N, sèche sur $MgSO_4$ et évapore sous vide le solvant. On obtient 1,8 g du produit attendu.

C) p-Toluènesulfonate de 4-(cyclopropylcarbonyl-N-méthylamino)-4-phényl pipéridine.

On hydrogène à TA et à pression atmosphérique un mélange de 1,8 g du composé obtenu à l'étape précédente, 0,85 g d'acide para-toluènesulfonique monohydrate, 0,35 g de palladium sur charbon à 10 % et 100 ml d'EtOH. On filtre le catalyseur sur Célite et évapore sous vide le filtrat. On obtient 1,5 g du produit attendu après cristallisation dans le mélange acétone/AcOEt.

PREPARATION 1.5

*p*-Toluènesulfonate de 4-phényl-4-(propionyl-N-méthylamino)pipéridine.

A) 4-(Acryloyl-N-méthylamino)-1-benzyl-4-phénylpipéridine.

On refroidit à 0°C une solution de 1,5 g du composé obtenu à l'étape A de la PREPARATION 1.4, 1,5 ml de triéthylamine dans 40 ml de DCM, ajoute goutte à goutte 0,5 ml de chlorure d'acryloyle et laisse sous agitation en laissant remonter la température à TA. On verse le mélange réactionnel dans l'eau, extrait au DCM, lave la phase organique à l'eau, par une solution de NaOH 2N, sèche sur $MgSO_4$ et évapore sous vide le solvant. On obtient 1,3 g du produit attendu après cristallisation dans le mélange éther/pentane.

B)*p*-Toluènesulfonate de 4-(acryloyl-N-méthylamino)-1-benzyl-4-phénylpipéridine.

A une solution de 1,15 g du composé obtenu à l'étape précédente dans 10 ml de DCM, on ajoute 0,59 g d'acide *p*-toluènesulfonique monohydrate et laisse en cristallisation. On obtient 1,65 g du produit attendu.

C) *p*-Toluènesulfonate de 4-phényl-4-(propionyl-N-méthylamino)pipéridine.

On hydrogène à TA et à pression atmosphérique un mélange de 1,64 g du composé obtenu à l'étape précédente, 0,2 g de palladium sur charbon à 10 % dans 100 ml d'EtOH. On filtre le catalyseur sur Célite® et évapore sous vide le solvant. On obtient 1,3 g du produit attendu.

PREPARATION 2.1

2-(3,4-Dichlorophényl)-5-(tétrahydropyran-2-yloxy)pentylamine.

A) 2-(3,4-Dichlorophényl)-5-(tétrahydropyran-2-yloxy)pentanenitrile.

15 g d'hydrure de sodium à 60 % dans l'huile sont mis en suspension dans 200 ml de THF anhydre. On ajoute goutte à goutte en 30 minutes une solution de 69,5 g de 3,4-dichlorophénylacétonitrile dans 500 ml de THF puis

on agite le mélange réactionnel à TA pendant 1 heure. Le mélange est refroidi à -20°C, on ajoute une solution de 85 g de 1-bromo-3-(tétrahydropyran-2-yloxy)propane dans 100 ml de THF. On laisse revenir le mélange à TA et après 2 heures, on le verse sur une solution de 50 g de chlorure d'ammonium dans 3 litres d'eau. On extrait à l'éther, lave avec une solution saturée de chlorure de sodium, décante, sèche sur $MgSO_4$ et concentre. Le résidu est chromatographié sur gel de silice en éluant par un mélange toluène et un gradient d'AcOEt (3 à 5 %). Les fractions de produit pur sont concentrées pour obtenir 77 g du produit attendu sous forme d'huile.

B) 2-(3,4-Dichlorophényl)-5-(tétrahydropyran-2-yloxy)pentylamine.

77 g de nitrile obtenu à l'étape précédente sont mis en solution dans 500 ml d'éthanol absolu. On ajoute 200 ml d'ammoniaque concentrée puis du nickel de Raney (10 % de la quantité de nitrile de départ). On hydrogène ensuite sous atmosphère d'hydrogène à TA et pression atmosphérique, 10,5 l d'hydrogène sont absorbés et le catalyseur séparé par filtration sur Célite. Le filtrat est concentré sous vide et le résidu est repris dans une solution de NaCl. Après extraction à l'éther et séchage par $MgSO_4$, on obtient 75 g du produit attendu sous forme d'huile.

PREPARATION 2.2

Chlorhydrate de 3-(3,4-dichlorophényl)-3-(3-hydroxypropyl)pipéridine.

A) 4-cyano-4-(3,4-dichlorophényl)heptanedioate de méthyle.

Dans un tricol, on dissout 37,2 g de 3,4-dichlorophénylacétonitrile et 34,43 g d'acrylate de méthyle dans 20 ml de dioxane ; on ajoute 1 ml de DBU, chauffe 2 heures à 60°C, évapore, dilue avec 400 ml d'acétate d'éthyle puis lave avec HCl dilué, une solution de NaCl, sèche sur $MgSO_4$ et évapore. Le produit attendu est cristallisé dans 100 ml d'acétate d'éthyle, et 100 ml d'éther avec 100 ml d'heptane. On obtient 47 g du produit.

B) 3-[5-(3,4-dichlorophényl)-2-oxopipérid-5-yl]propionate de méthyle.

On dissout 40 g du composé préparé à l'étape A dans 500 ml de 2-méthoxyéthanol, on ajoute 2 g de Nickel de Raney et hydrogène à 40°C sous pression atmosphérique pendant 3 jours. On filtre, évapore, et obtient le produit attendu sous forme d'huile (39 g).

C) Acide 3-[5-(3,4-dichlorophényl)-2-oxopipérid-5-yl]propanoïque.

On dissout 17 g du composé préparé à l'étape précédente dans 250 ml de méthanol, ajoute 2,8 g de potasse et 10 ml d'eau puis on porte à reflux pendant 2 heures. On évapore à sec, reprend l'huile obtenue par 200 ml d'eau et extrait par 100 ml d'acétate d'éthyle. La phase aqueuse est acidifiée par une solution d'HCl à 30 % puis on filtre et sèche le précipité formé. On recristallise dans le méthanol à chaud et obtient 18,3 g du composé attendu.

D) Chlorhydrate de 3-(3,4-dichlorophényl)-3-(3-hydroxypropyl)pipéridine.

On dissout 5 g du composé obtenu à l'étape précédente dans 20 ml de THF, ajoute 75 ml de borane (concentration 1 M dans le THF) et chauffe au reflux pendant 24 heures, sous azote. On ajoute 25 ml de méthanol, 50 ml d'HCl 4N et laisse sous agitation 30 minutes puis on ajoute de la soude à 40 % jusqu'à un pH supérieur à 10. On extrait 3 fois par 150 ml de DCM, sèche la phase organique sur $MgSO_4$ et évapore. Le résidu est mis en solution dans DCM avec une solution 4N d'HCl dans l'éther. Après évaporation, on obtient une mousse et le produit attendu (4,5 g) cristallise dans le mélange AcOEt/éther;

EXEMPLE 1

Chlorhydrate de 1-benzoyl-3-(3,4-dichlorophényl)-3-[3-(4-pivaloylamino-4-phénylpipérid-1-yl)propyl]pipéridine, isomère (+).

A) Chlorhydrate de 3-(3,4-dichlorophényl)-3-(3-hydroxypropyl)pipéridine, isomère (+).

On dissout 10 g du composé obtenu à la PREPARATION 2.2 dans 20 ml d'eau, ajoute 5 ml de soude à 40 %, extrait 3 fois par 50 ml de DCM, sèche la phase organique sur $MgSO_4$ et évapore pour obtenir 9 g d'huile. 2,7 g de l'huile obtenue sont dissous dans 50 ml d'isopropanol, on ajoute 2,36 g d'acide 10-camphosulfonique, isomère +, à chaud et on laisse refroidir. Les cristaux formés (3,86 g) sont dissous dans NaOH à 10 %, on extrait au chloroforme, sèche sur $MgSO_4$ et évapore. On obtient 2,3 g du produit sous forme d'huile dont on fait le chlorhydrate. On mesure le pouvoir rotatoire du chlorhydrate.

$$[\alpha]_D^{25} = +5,5° \text{ (c = 0,1 ; méthanol).}$$

Une deuxième cristallisation effectuée à partir de 2,12 g de l'huile obtenue et 1,84 g d'acide camphosulfonique (isomère +) procure 3,27 g de cristaux qui, après basification par la soude et extraction, donnent 2,10 g du produit attendu sous forme d'huile dont on fait le chlorhydrate.

$$[\alpha]_D^{25} = + 6,5° \text{ (c = 0,1 ; méthanol).}$$

Après une troisième cristallisation, on obtient le même pouvoir rotatoire. La pureté chirale mesurée par HPLC chirale est supérieur à 98 %.

B) N-Boc-3-(3,4-dichlorophényl)-3-(3-hydroxypropyl]pipéridine, isomère (+).

Dans 100 ml de DCM, on dissout 900 mg du composé préparé à l'étape précédente, 600 mg de TEA et 610 mg de $(Boc)_2O$ et on laisse réagir sous azote, à TA pendant 30 minutes. On évapore, dissout le résidu dans AcOEt, lave avec un tampon à pH = 2, par de la soude diluée, une solution de NaCl, puis on sèche sur $MgSO_4$ et évapore pour obtenir le produit attendu sous forme d'une huile (1,1 g).

C) N-Boc-3-(3,4-dichlorophényl)-3-(3-mésyloxypropyl)pipéridine, isomère (+).

1,1 g du composé obtenu à l'étape précédente sont dissous à 0°C sous azote dans 10 ml de DCM, on ajoute 700 mg de TEA et 325 mg de chlorure de mésyle dissous dans 3 ml de DCM. Après 2 heures sous agitation, on évapore, reprend l'huile résiduelle dans AcOEt. lave par une solution tampon à pH 2, de l'eau, une solution de NaCl, puis on sèche et évapore. On obtient 1,4 g du produit attendu sous forme d'huile.

D) N-Boc-3-(3,4-dichlorophényl)-3-[3-(4-pivaloylamino-4-phénylpipérid-1-yl) propyl]pipéridine, isomère (+).

Dans 25 ml d'acétonitrile, on dissout 1,4 g du composé préparé à l'étape précédente et 900 mg de 4-phényl-4-pivaloylaminopipéridine obtenu à la PREPARATION 1.1. On ajoute 450 mg de $K_2CO_3$ et laisse sous agitation pendant 2 heures à 60°C. On évapore, dissout dans AcOEt, lave par une solution tampon à pH 2, une solution de NaOH diluée, une solution de NaCl, sèche sur $MgSO_4$ et évapore pour obtenir 1,65 g du produit attendu sous forme d'une huile.

E) Chlorhydrate de 3-(3,4-dichlorophényl)-3-[3-(4-pivaloylamino-4-phényl-pipérid-1-yl)propyl]pipéridine, isomère (+).

Dans 25 ml de DCM, on ajoute 1,65 g du composé préparé à l'étape précédente et 5 ml d'HCl de concentration 4N dans l'éther. Après 1 heure d'agitation on obtient 1,12 g du composé attendu.

F) Chlorhydrate de 1-benzoyl-3-(3,4-dichlorophényl)-3-[3-(4-pivaloylamino-4-phénylpipérid-1-yl)propyl]pipéridine, isomère (+).

Dans 15 ml de DCM, on dissout 1,10 g du composé obtenu à l'étape précédente, 800 mg de TEA et 252 mg de chlorure de benzoyle, sous azote, à 0°C. Après 15 minutes, on évapore, dissout dans AcOEt, lave par une solution d'HCl dilué, par une solution de NaCl, sèche sur $MgSO_4$ et évapore. La mousse obtenue est chromatographiée sur silice en éluant par un mélange DCM/MeOH en gradient jusqu'à 5 %. Le produit obtenu est salifié par une solution d'éther chlorhydrique.

$$[\alpha]_D^{25} = +24,3 \text{ (c = 0,1 ; méthanol).}$$

EXEMPLE 2

Chlorhydrate de 1-benzoyl-3-(3,4-dichlorophényl)-3-[3-(4-pivaloylamino-4-phényl pipérid-1-yl)propyl]pipéridine, isomère (-).

Ce composé est obtenu comme à l'EXEMPLE 1 en utilisant l'acide camphosulfonique, isomère (-) à l'étape A.

EXEMPLE 3

Chlorhydrate de 1-benzoyl-3-(3,4-dichlorophényl)-3-[3-(4-pivaloylamino-4-phénylpipérid-1-yl)propyl]pipéridine, racémique.

Ce composé est obtenu comme à l'EXEMPLE 1 sans effectuer la résolution optique de l'étape A.

EXEMPLE 4

Chlorhydrate de 1-benzoyl-3-(3,4-dichlorophényl)-3-[3-[4-(acétyl-N-méthylamino)-4-phénylpipérid-1-yl]propyl]pipéridine, monohydrate.

A) N-Boc-3-(3,4-dichlorophényl)-3-(3-hydroxypropyl)pipéridine.

On laisse 1 heure sous agitation, à TA et sous atmosphère d'azote, un mélange de 23 g du composé obtenu à la PREPARATION 2.2, 15 g de triéthylamine, 16 g de $(Boc)_2O$ dans 100 ml de DCM. On concentre sous vide le mélange réactionnel, extrait le résidu à l'AcOEt, lave par une solution tampon pH = 2, par une solution de NaOH

à 5 %, par une solution saturée de NaCl, sèche sur $MgSO_4$ et évapore sous vide le solvant. On obtient 30 g du produit attendu sous forme d'huile.

B) N-Boc-3-(3,4-dichlorophényl)-3-(3-mésyloxypropyl)pipéridine.

On refroidit à 0°C une solution de 30 g du composé obtenu à l'étape précédente, 15 ml de triéthylamine dans 200 ml de DCM et ajoute, sous atmosphère d'azote et goutte à goutte, une solution de 9 g de chlorure de mésyle dans 50 ml de DCM. On laisse 2 heures sous agitation et évapore sous vide le solvant. On extrait le résidu à l'AcOEt, lave par une solution tampon pH = 2, par une solution de NaOH à 5 %, par une solution saturée de NaCl, sèche sur $MgSO_4$ et évapore sous vide le solvant. On obtient 34 g du produit attendu.

C) N-Boc-3-(3,4-dichlorophényl)-3-[3-[4-(acétyl-N-méthylamino)-4-phénylpipérid-1-yl]propyl]pipéridine.

On chauffe à 60°C pendant 3 heures un mélange de 1 g du composé obtenu à l'étape précédente, 2 g du composé obtenu à la PREPARATION 1.3, 0,6 g de $K_2CO_3$ dans 15 ml de DMF puis on laisse une nuit sous agitation à TA. On ajoute au mélange réactionnel de l'AcOEt, lave la phase organique à l'eau, par une solution saturée de NaCl, sèche sur $MgSO_4$ et évapore sous vide le solvant. On chromatographie le résidu sur silice H en éluant par le mélange DCM/MeOH (95/5 ; v/v). On obtient 0,78 g du produit attendu.

D) Chlorhydrate de 3-(3,4-dichlorophényl)-3-[3-[4-(acétyl-N-méthylamino)-4-phényl pipérid-1-yl]propyl]pipéridine.

A une solution de 0,78 g du composé obtenu à l'étape précédente dans 5 ml de DCM on ajoute 2 ml d'une solution saturée de HCl dans l'éther et laisse 1 heure sous agitation à TA. On évapore sous vide le mélange réactionnel et obtient 0,8 g du produit attendu que l'on utilise tel quel à l'étape suivante.

E) Chlorhydrate de 1-benzoyl-3-(3,4-dichlorophényl)-3-[3-[4-(acétyl-N-méthyl amino)-4-phénylpipérid-1-yl]propyl] pipéridine, monohydrate.

On refroidit à 0°C, un mélange de 0,75 g du composé obtenu à l'étape précédente, 0,6 ml de triéthylamine dans 10 ml de DCM et ajoute, sous atmosphère d'azote, 0,18 g de chlorure de benzoyle. On laisse 2 heures sous agitation à 0°C et concentre sous vide le mélange réactionnel. On extrait le résidu à l'AcOEt, lave par une solution tampon pH = 2, par une solution à 5 % de NaOH, par une solution saturée de NaCl, sèche sur $MgSO_4$ et évapore sous vide le solvant. On chromatographie le résidu sur silice H en éluant par le mélange DCM/MeOH (95/5 ; v/v). On reprend le produit obtenu au DCM, acidifie à pH = 1 par ajout d'éther chlorhydrique et évapore sous vide. On obtient 0,5 g du chlorhydrate attendu, F = 171°C.

EXEMPLE 5

Chlorhydrate de 1-benzoyl-3-(3,4-dichlorophényl)-3-[3-[4-(acétyl-N-méthyl amino)-4-phénylpipérid-1-yl]propyl]pipéridine, monohydrate, isomère (+).

A) 1-Benzoyl-3-(3,4-dichlorophényl)-3-(3-hydroxypropyl)pipéridine, isomère (+).

On refroidit à -20°C une solution de 7,45 g du composé obtenu à l'étape A de l'EXEMPLE 1 (sous forme de base) dans 30 ml de DCM et ajoute 4 ml de triéthylamine puis, goutte à goutte, 2,85 ml de chlorure de benzoyle. On laisse sous agitation en laissant remonter la température à TA puis lave le mélange réactionnel par une solution d'HCl 0,5N, par une solution saturée de $Na_2CO_3$, sèche la phase organique sur $MgSO_4$ et évapore sous vide le solvant. On chromatographie le résidu sur silice en éluant par le gradient du mélange DCM/AcOEt de (90/10 ; v/v) jusqu'à (80/20 ; v/v) puis par DCM/MeOH (97/3 ; v/v). On obtient 9,40 g du produit attendu.

B) 1-Benzoyl-3-(3,4-dichlorophényl)-3-(3-mésyloxypropyl)pipéridine, isomère (+).

On refroidit à -10°C une solution de 9,4 g du composé obtenu à l'étape précédente, 5 ml de triéthylamine dans 50 ml de DCM et ajoute, goutte à goutte, 2,24 ml de chlorure de mésyle. On laisse sous agitation en laissant remonter la température à TA et concentre sous vide le solvant. On extrait le résidu à l'AcOEt, lave deux fois à l'eau, par une solution saturée de NaCl, sèche sur $MgSO_4$ et évapore sous vide le solvant. On obtient 10 g du produit attendu.

C) Chlorhydrate de 1-benzoyl-3-(3,4-dichlorophényl)-3-[3-[4-(acétyl-N-méthyl amino)-4-phénylpipérid-1-yl]propyl] pipéridine, monohydrate, isomère (+).

On chauffe à 100°C pendant 2 heures, sous atmosphère d'azote, un mélange de 2 g du composé obtenu à l'étape précédente, 4 g de p-toluènesulfonate de 4-(acétyl-N-méthylamino)-4-phénylpipéridine, 1 g de $K_2CO_3$ dans 50 ml d'acétonitrile et 50 ml de DMF. On concentre sous vide le mélange réactionnel, extrait le résidu à l'AcOEt, lave à l'eau, par une solution d'HCl 0,5N, par une solution de NaOH à 10 %, par une solution saturée de NaCl, sèche sur $MgSO_4$ et évapore sous vide le solvant. On chromatographie le résidu sur silice H en éluant par le gradient du mélange DCM/MeOH de (99/1 ; v/v) jusqu'à (95/5 ; v/v). On reprend le produit obtenu au DCM, acidifie à pH = 1 par ajout d'éther chlorhydrique et évapore sous vide. On obtient 1,05 g du produit attendu.

$$[\alpha]_D^{25} = +21,5° \pm 0,5 \text{ (c = 1 ; MeOH).}$$

EXEMPLE 6

Chlorhydrate de 1-benzoyl-3-(3,4-dichlorophényl)-3-[3-[4-(acétyl-N-méthyl amino)-4-phénylpipérid-1-yl]propyl]pipéridine, monohydrate, isomère (-).

A) Chlorhydrate de 3-(3,4-dichlorophényl)-3-(3-hydroxypropyl)pipéridine, isomère (-).

A une solution de 4,7 g du composé obtenu à la PREPARATION 2.2, sous forme de base libre, dans 100 ml d'isopropanol on ajoute 3,8 g d'acide 10-camphosulfonique, isomère (-), et chauffe à reflux. Après refroidissement, cristallisation et essorage des cristaux formés (4,90 g), on dissout ces derniers dans une solution de NaOH à 10 %, extrait au chloroforme, sèche sur $MgSO_4$ et évapore sous vide le solvant. On obtient 2,7 g du produit sous forme d'huile dont on fait le chlorhydrate. On mesure le pouvoir rotatoire du chlorhydrate.

$$[\alpha]_D^{25} = 6,5^\circ \text{ (c = 1 ; MeOH).}$$

Une deuxième cristallisation est effectuée à partir de 2,6 g de l'huile obtenue, 2,77 g d'acide 10-camphosulfonique isomère (-) et 40 ml d'isopropanol. Après basification par la soude, extraction au chloroforme, sèchage sur $MgSO_4$ et évaporation, on obtient le produit attendu sous forme d'huile dont on fait le chlorhydrate. On obtient 2,4 g du produit attendu.

$$[\alpha]_D^{25} = -6,8^\circ \text{ (c = 1 ; MeOH).}$$

B) 1-Benzoyl-3-(3,4-dichlorophényl)-3-(3-hydroxypropyl)pipéridine, isomère (-).

On refroidit à -30°C une solution de 11 g du composé obtenu à l'étape précédente (sous forme de base libre), 6 ml de triéthylamine dans 75 ml de DCM et ajoute, goutte à goutte, 4,2 ml de chlorure de benzoyle. On laisse sous agitation en laissant remonter la température à TA puis verse dans l'eau le mélange réactionnel. On extrait au DCM, lave la phase organique à l'eau, par une solution de NaOH 0,5N, sèche sur $MgSO_4$ et évapore sous vide le solvant. On chromatographie le residu sur silice en éluant par le gradient du mélange DCM/AcOEt de (85/15 ; v/v) jusqu'à (75/25 ; v/v) puis par DCM/MeOH (97/3 ; v/v). On obtient 10 g du produit attendu.

C) 1-Benzoyl-3-(3,4-dichlorophényl)-3-(3-mésyloxypropyl)pipéridine, isomère (-).

On refroidit à -30°C une solution de 10 g du composé obtenu à l'étape précédente, 5,5 ml de triéthylamine dans 100 ml de DCM et ajoute, goutte à goutte, 2,4 ml de chlorure de mésyle. On laisse sous agitation en laissant remonter la température à TA et concentre sous vide. On extrait le résidu à l'AcOEt, lave deux fois à l'eau, par une solution saturée de NaCl, sèche sur $MgSO_4$ et évapore sous vide. On obtient 11 g du produit attendu.

D) Chlorhydrate de 1-benzoyl-3-(3,4-dichlorophényl)-3-[3-[4-(acétyl-N-méthyl amino)-4-phénylpipérid-1-yl]propyl] pipéridine, monohydrate, isomère (-).

On prépare ce composé selon le mode opératoire décrit à l'étape C de l'EXEMPLE 5 à partir de 0,5 g du composé obtenu à l'étape précédente et 0,8 g de p-toluènesulfonate de 4-(acétyl-N-méthylamino)-4-phénylpipéridine. On obtient 0,375 g du produit attendu.

$$[\alpha]_D^{25} = -21,5^\circ \pm 0,5 \text{ (c = 1 ; MeOH).}$$

Selon un mode opératoire semblable à celui décrit dans l'exemple 1, on prépare les composés selon l'invention décrits dans le tableau 1 ci-dessous :

TABLEAU 1

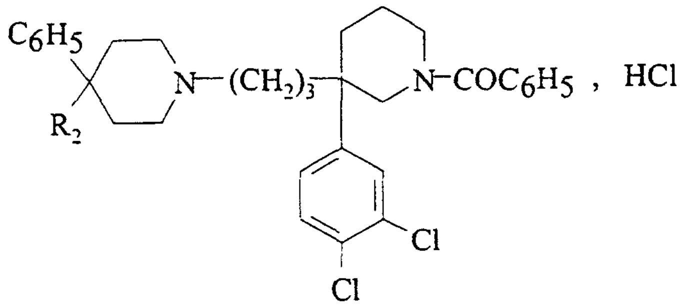

| Exemples | $R_2$ | F °C |
|---|---|---|
| 7 | $-NHCOC_2H_5$ | 187 |
| 8 | $-NHCOCH(CH_3)_2$ | 170 |
| 9 | $-NCH_3COiPr$ | 192 |
| 10 | -NHCOBu | 170 |

EXEMPLE 11

Chlorhydrate de 1-(4-iodobenzoyl)-3-(3,4-dichlorophényl)-3-[3-[4-(acétyl-N-méthylamino)-4-phénylpipérid-1-yl]propyl]pipéridine, monohydrate, isomère (+).

A) 1-(4-Iodobenzoyl)-3-(3,4-dichlorophényl)-3-(3-hydroxypropyl)pipéridine, isomère (+).

A un mélange de 5 g du composé obtenu à l'étape A de l'EXEMPLE 1, 3,9 g d'acide 4-iodobenzoïque dans 100 ml de DCM on ajoute à TA 6,5 ml de triéthylamine puis 8,2 g de BOP et laisse 15 minutes sous agitation à TA. On concentre sous vide le mélange réactionnel, reprend le résidu à l'éther, lave la phase organique à l'eau, par une solution de NaOH 1N, à l'eau, par une solution de HCl 1N, par une solution saturée de NaCl, sèche sur $MgSO_4$ et évapore sous vide le solvant. On chromatographie le résidu sur silice en éluant au DCM puis par le mélange DCM/AcOEt (50/50 ; v/v) puis à l'AcOEt. On obtient 8,5 g du produit attendu.

B) 1-(4-Iodobenzoyl)-3-(3,4-dichlorophényl)-3-(3-mésyloxypropyl)pipéridine, isomère (+).

On refroidit à 0°C une solution de 8,5 g du composé obtenu à l'étape précédente, 2,7 ml de triéthylamine dans 150 ml de DCM et ajoute, goutte à goutte, 1,5 ml de chlorure de mésyle. On laisse sous agitation en laissant remonter la température à TA et concentre sous vide. On extrait le résidu à l'éther, lave deux fois la phase organique à l'eau, sèche sur $MgSO_4$ et évapore sous vide le solvant. On obtient 10 g du produit attendu.

C) Chlorhydrate de 1-(4-iodobenzoyl)-3-(3,4-dichlorophényl)-3-[3-[4-acétyl-N-méthylamino)-4-phénylpipérid-1-yl]propyl]pipéridine, monohydrate, isomère (+).

On chauffe à 80°C pendant 1 heure un mélange de 10 g du composé obtenu à l'étape précédente, 8,2 g de *p*-toluènesulfonate de 4-(acétyl-N-méthylamino)-4-phénylpipéridine, 9,3 g de $K_2CO_3$ dans 80 ml de DMF. Puis on ajoute 4 g de *p*-toluènesulfonate de 4-(acétyl-N-méthylamino)-4-phénylpipéridine au mélange réactionnel et poursuit le chauffage à 80°C pendant 2 heures. Après refroidissement, on verse le mélange réactionnel dans de l'eau glacée, essore le précipité formé et le lave à l'eau. On dissout le précipité dans du DCM, sèche la phase organique sur $MgSO_4$ et évapore sous vide le solvant. On chromatographie le résidu sur silice H en éluant au DCM puis par le mélange DCM/MeOH (95/5 ; v/v). On reprend le produit obtenu au DCM, acidifie à pH = 1 par ajout d'éther

chlorhydrique et évapore sous vide. On obtient 9,2 g du produit attendu après cristallisation dans l'éther, F = 172°C (déc).

$$[\alpha]_D^{25} = +27,2° \ (c = 1 ; MeOH).$$

**Revendications**

1. Composé choisi parmi les composés ci-après:

   la 1-benzoyl-3-(3,4-dichlorophényl)-3-[3-(4-(acétyl-N-méthylamino)-4-phényl-pipérid-1-yl)propyl]pipéridine,
   la 1-benzoyl-3-(3,4-dichlorophényl)-3-[3-(4-propionylamino-4-phénylpipérid-1-yl)propyl]pipéridine,
   la 1-benzoyl-3-(3,4-dichlorophényl)-3-[3-(4-(propionyl-N-méthylamino)-4-phényl-pipérid-1-yl)propyl]pipéridine,
   la 1-benzoyl-3-(3,4-dichlorophényl)-3-[3-(4-butyrylamino-4-phénylpipérid-1-yl)-propyl]pipéridine,
   la 1-benzoyl-3-(3,4-dichlorophényl)-3-[3-(4-(butyryl-N-méthylamino)-4-phényl-pipérid-1-yl)propyl]pipéridine,
   la 1-benzoyl-3-(3,4-dichlorophényl)-3-[3-(4-isobutyrylamino-4-phénylpipérid-1-yl)-propyl]pipéridine,
   la 1-benzoyl-3-(3,4-dichlorophényl)-3-[3-(4-(isobutyryl-N-méthylamino)-4-phényl-pipérid-1-yl)propyl]pipéridine,
   la 1-benzoyl-3-(3,4-dichlorophényl)-3-[3-(4-valérylamino-4-phénylpipérid-1-yl)-propyl]pipéridine,
   la 1-benzoyl-3-(3,4-dichlorophényl)-3-[3-(4-(valéryl-N-méthylamino)-4-phényl-pipérid-1-yl)propyl]pipéridine,
   la 1-benzoyl-3-(3,4-dichlorophényl)-3-[3-(4-isovalérylamino-4-phénylpipérid-1-yl)propyl]pipéridine,
   la 1-benzoyl-3-(3,4-dichlorophényl)-3-[3-(4-(isovaléryl-N-méthylamino)-4-phényl-pipérid-1-yl)propyl]pipéridine,
   la 1-benzoyl-3-(3,4-dichlorophényl)-3-[3-(4-pivaloylamino-4-phénylpipérid-1-yl)-propyl]pipéridine,
   la 1-benzoyl-3-(3,4-dichlorophényl)-3-[3-(4-(pivaloyl-N-méthylamino)-4-phényl-pipérid-1-yl)propyl]pipéridine,

   sous forme d'un racémate ou d'un énantiomère (+) ou (-),
   et leurs sels pharmaceutiquement acceptables.

2. Composé selon la revendication 1, qui est la 1-benzoyl-3-(3,4-dichlorophényl)-3-[3-(4-(acétyl-N-méthylamino)-4-phénylpipérid-1-yl)propyl]-pipéridine sous forme d'isomère (+).

3. Composé selon la revendication 1, qui est le chlorhydrate de 1-benzoyl-3-(3,4-dichlorophényl)-3-[3-(4-(acétyl-N-méthylamino)-4-phénylpipérid-1-yl)propyl]-pipéridine sous forme d'isomère (+).

4. Procédé pour la préparation d'un composé de formule (I) selon la revendication 1 et de ses sels, **caractérisé en ce que**:

   a) on traite un composé de formule :

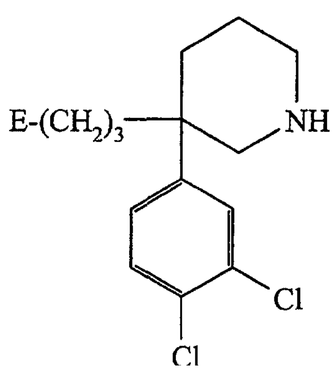

1

dans laquelle E représente un hydroxyle ou éventuellement un groupe O-protégé tel que par exemple un tétrahydropyran-2-yloxy, un benzoyloxy ou un $(C_1\text{-}C_4)$alkylcarbonyloxy ou un groupe

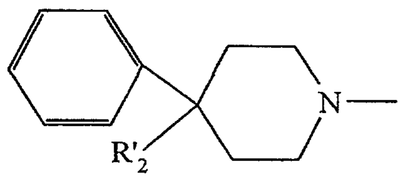

2

dans lequel R'$_2$ représente un groupe acétyl-N-méthylamino, propionylamino, propionyl-N-méthylamino, butyrylamino, butyryl-N-méthylamino, isobutyrylamino, isobutyryl-N-méthylamino, valérylamino, valéryl-N-méthylamino, isovalérylamino, isovaléryl-N-méthylamino, pivaloylamino ou pivaloyl-N-méthylamino ou un précurseur d'un tel groupe,
avec un dérivé fonctionnel d'un acide de formule :

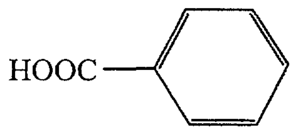

3

pour obtenir un composé de formule :

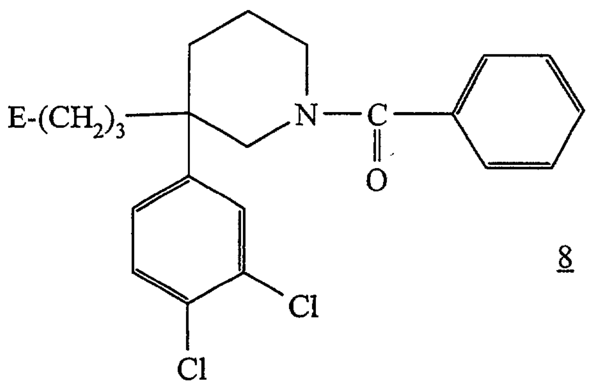

8

b) on élimine éventuellement le groupe O-protecteur par action d'un acide ou d'une base,
c) on traite l'alcool ainsi obtenu de formule :

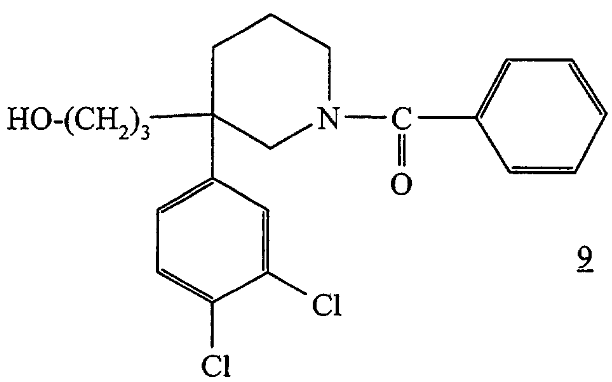

9

avec un composé de formule :

$W\text{-}SO_2\text{-}Cl$ 9a

dans laquelle W représente un groupe méthyle, phényle, tolyle ou trifluorométhyle,
d) on fait réagir le sulfonate ainsi obtenu de formule :

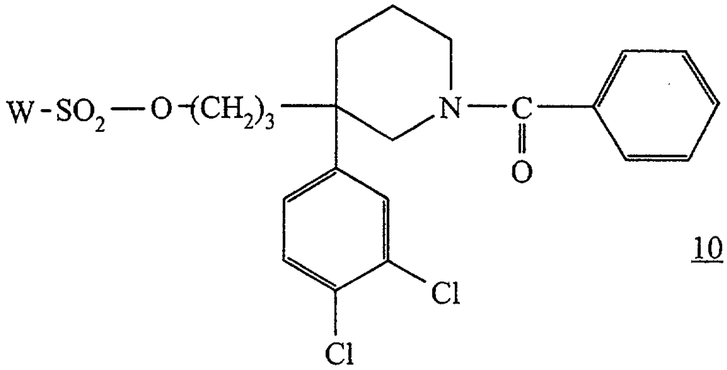

10

avec une amine secondaire de formule :

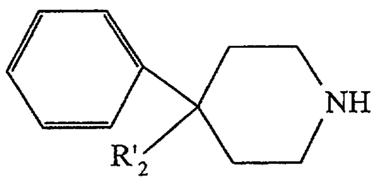

11

dans laquelle $R'_2$ est tel que défini ci-dessus,
e) après déprotection éventuelle de l'hydroxyle ou de l'amino représenté par $R'_2$, on transforme éventuellement le produit obtenu en l'un de ses sels.

**5.** Procédé pour la préparation d'un composé de formule (I) selon la revendication 1 et de ses sels, **caractérisé en ce que**

a1) on protège la fonction amine du composé de formule :

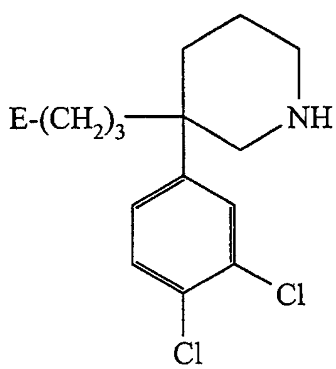

1

dans laquelle E représente un hydroxyle ou éventuellement un groupe O-protégé tel que par exemple un tétrahydropyran-2-yloxy, un benzoyloxy ou un $(C_1\text{-}C_4)$alkylcarbonyloxy ou un groupe

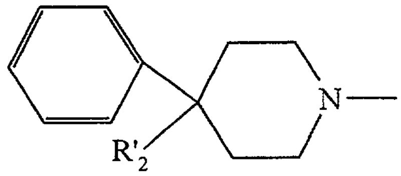

2

dans lequel $R'_2$ est tel que défini dans la revendication 4,
par un groupe protecteur de façon à préparer un composé de formule :

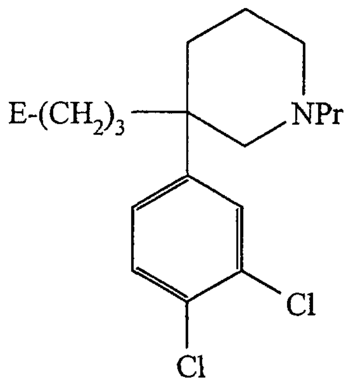

12

dans laquelle Pr représente un groupe protecteur de l'azote par exemple un *tert*-butoxycarbonyle (Boc), un trityle ou un benzyle,
b1) on élimine éventuellement le groupe O-protecteur par action d'un acide ou d'une base,
c1) on traite l'alcool ainsi obtenu de formule :

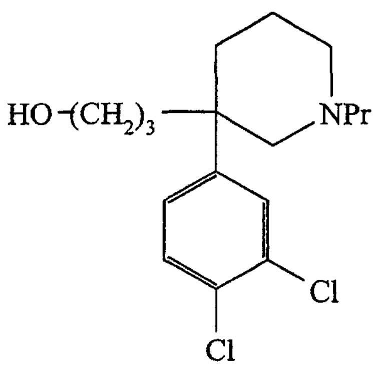

13

avec un composé de formule :

$$W - SO_2 - Cl \qquad \underline{9a}$$

dans laquelle W représente un groupe méthyle, phényle, tolyle ou trifluorométhyle,
d1) on fait réagir le sulfonate ainsi obtenu de formule :

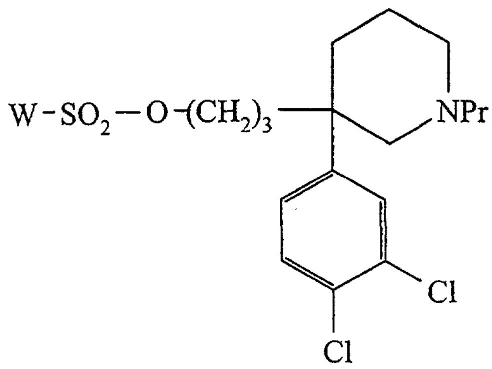

14

avec une amine secondaire :

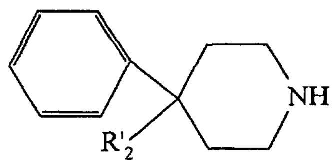

11

dans laquelle R'$_2$ est tel que défini ci-dessus,
pour obtenir le composé de formule:

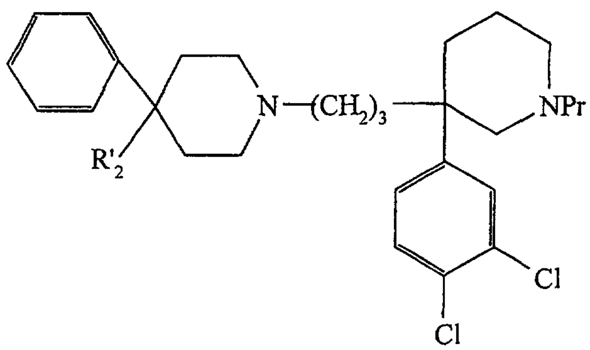

15

e1) on effectue la déprotection de l'azote en milieu acide,
f1) on traite le composé ainsi obtenu de formule :

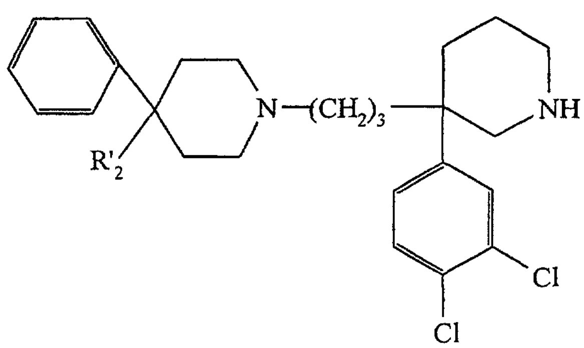

16

par le composé 3 défini dans la revendication 4,
g1) après déprotection éventuelle de l'hydroxyle ou de l'amino représenté par R'$_2$, on transforme éventuellement le produit obtenu de formule (I) en l'un de ses sels.

**6.** Composition pharmaceutique contenant à titre de principe actif un composé selon l'une des revendications 1 à 3.

**7.** Composition pharmaceutique selon la revendication 6, sous forme d'unité de dosage, dans laquelle le principe actif est mélangé à au moins un excipient pharmaceutique.

**8.** Composition pharmaceutique selon la revendication 7 contenant de 0,5 à 1000 mg de principe actif.

**9.** Composition pharmaceutique selon la revendication 8 contenant de 2,5 à 250 mg de principe actif.

**10.** Utilisation d'un composé selon l'une des revendications 1 à 3 ou d'un de ses sels pharmaceutiquement acceptables, pour la préparation d'un médicament destiné à combattre les maladies psychiatriques, les maladies d'origine psychosomatique, l'hypertension, ou les pathologies liées à des désordres de neurotransmission ou neuromodulation $NK_B$ dépendante.

**11.** Utilisation selon la revendication 10, dans laquelle ledit composé est le chlorhydrate de 1-benzoyl-3-(3,4-dichlorophényl)-3-[3-(4-(acétyl-N-méthylamino)-4-phénylpipérid-1-yl)propyl]pipéridine sous forme d'isomère (+).

**Patentansprüche**

**1.** Verbindung ausgewählt aus den nachstehenden Verbindungen:

1-Benzoyl-3-(3,4-dichlorphenyl)-3-[3-(4-(acetyl-N-methylamino)-4-phenylpiperid-I-yl)-propyl]piperidin,
1-Benzoyl-3-(3,4-dichlorohenyl)-3-[3-(4-propionylamino-4-phenylpiperid-I-yl)propyl]piperidin,
1-Benzoyl-3-(3,4-dichlorphenyl)-3-[3-(4-(propionyl-N-methylamino)-4-phenylpiperid-I-yl)-propyl]piperidin,
1-Benzoyl-3-(3,4-dichlorphenyl)-3-(3-(4-butyrylamino-4-phenylpiperid-I-yl)propyl]piperidin,
1-Benzoyl-3-(3,4-dichlorphenyl)-3-[3-(4-(butyryl-N-methylamino)-4-phenylpiperid-I-yl)-propyl]piperidin,
1-Benzoyl-3-(3,4-dichlorphenyl)-3-[3-(4-isobutyrylamino-4-phenylpiperid-I-yl)propyl]piperidin,
1-Benzoyl-3-(3,4-dichlorphenyl)-3-[3-(4(isobutyryl-N-methylamino)-4-phenylpiperid-I-yl)-propyl]piperidin,
1-Benzoyl-3-(3,4-dichlorphenyl)-3-[3-(4-valerylamino-4-phenylpiperid-I-yl)propyl]piperidin,
1-Benzoyl-3-(3,4-dichlorphenyl)-3-[3-(4-(valeryl-N-methylamino)4-phenylpiperid-I-yl)-propyl]piperidin,
1-Benzoyl-3-(3,4-dichlorphenyl)-3-[3-(4-isovalerylamino-4-phenylpiperid-I-yl)propyl]piperidin,
1-Benzoyl-3-(3,4-dichlorphenyl)-3-[3-(4-(isovaleryl-N-methylamino)-4-phenylpiperid-I-yl)-propyl]piperidin,
1-Benzoyl-3-(3,4-dichlorphenyl)-3-[3-(4-pivaloylamino-4-phenylpiperid-I-yl)propyl]piperidin,
1-Benzoyl-3-(3,4-dichlorphenyl)-3-[3-(4-(pivaloyl-N-methylamino)-4-phenylpiperid-I-yl)-propyl]piperidin

in Form eines Racemats oder eines (+)- oder (-)-Enantiomers und die pharmazeutisch annehmbaren Salze derselben.

**2.** Verbindung nach Anspruch 1, und zwar 1-Benzoyl-3-(3,4-dichlorphenyl)-3-[3-(4-(acetyl-N-methylamino)-4-phenylpiperid-I-yl)propyl]piperidin in der (+)-Isomerform.

**3.** Verbindung nach Anspruch 1, und zwar 1-Benzoyl-3-(3,4-dichlorphenyl)-3-[3-(4-(acetyl-N-methylamino)-4-phenylpiperid-I-yl)propyl]piperidin-hydrochlorid in der (+)-Isomerform.

**4.** Verfahren zur Herstellung einer Verbindung der Formel (I) nach Anspruch 1 und ihrer Salze, **dadurch gekennzeichnet**, dass

a) eine Verbindung der Formel:

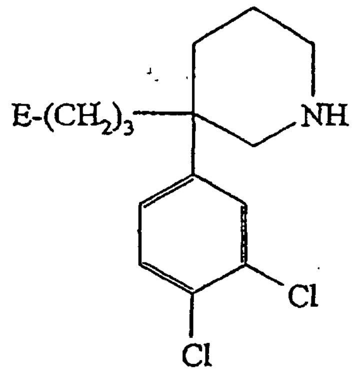

1

worin E Hydroxyl oder gegebenenfalls eine O-geschützte Gruppe darstellt, wie beispielsweise Tetrahydropyran-2-yloxy, Benzoyloxy oder ($C_1$-$C_4$)-Alkylcarbonyloxy oder eine Gruppe:

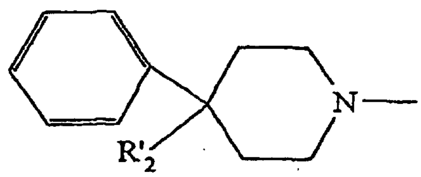

2

worin $R'_2$ Acetyl-N-methylamino, Propionylamino, Propionyl-N-methylamino, Butyrylamino, Butyryl-N-methylamino, Isobutyrylamino, Isobutyryl-N-methylamino, Valerylamino, Valeryl-N-methylamino, Isovalerylamino, Isovaleryl-N-methylamino, Pivaloylamino oder Pivaloyl-N-methylamino oder einen Vorläufer einer solchen Gruppe darstellt,
mit einem funktionellen Säurederivat der Formel:

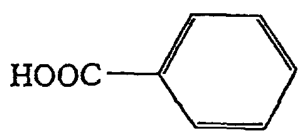

3

behandelt wird, um eine Verbindung der Formel:

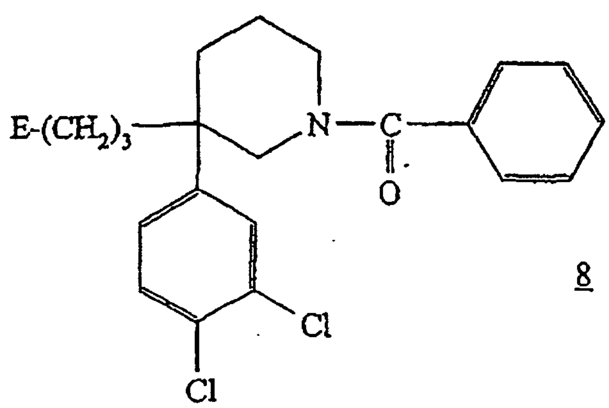

8

zu erhalten,
b) die O-Schutzgruppe durch Einwirken einer Säure oder einer Base gegebenenfalls entfernt wird,

c) der so erhaltene Alkohol der Formel

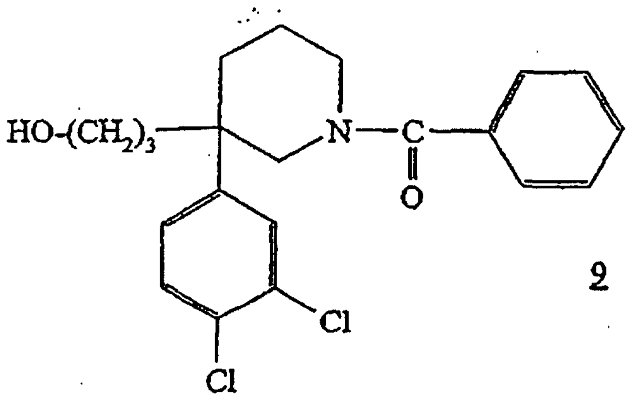

9

mit einer Verbindung der Formel:

$W\text{-}SO_2\text{-}Cl$ 9a

worin W Methyl, Phenyl, Tolyl oder Trifluormethyl darstellt, behandelt wird,
d) das so erhaltene Sulfonat der Formel:

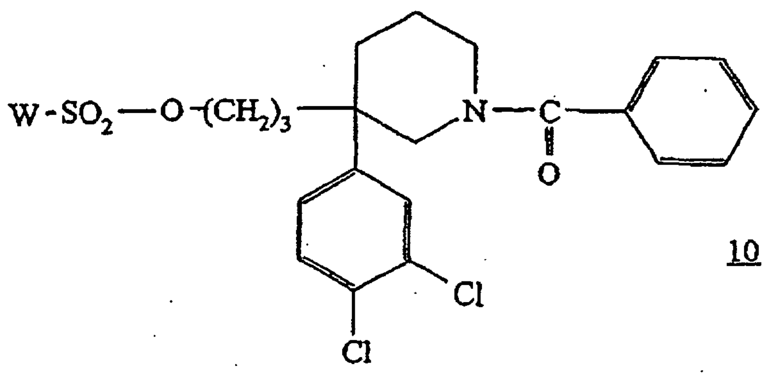

10

mit einem sekundären Amin der Formel:

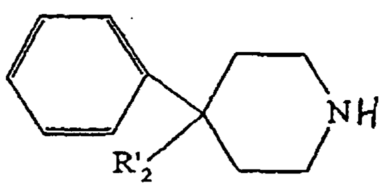

11

worin $R'_2$ wie oben definiert ist, zur Umsetzung gebracht wird,
e) nach gegebenenfalls Entschützen des durch $R'_2$ dargestellten Hydroxyls oder Aminos das erhaltene Produkt gegebenenfalls in eines seiner Salze übergeführt wird.

**5.** Verfahren zur Herstellung einer Verbindung der Formel (I) nach Anspruch 1 und ihrer Salze, **dadurch gekennzeichnet**, dass

a1) die Aminfunktion der Verbindung der Formel:

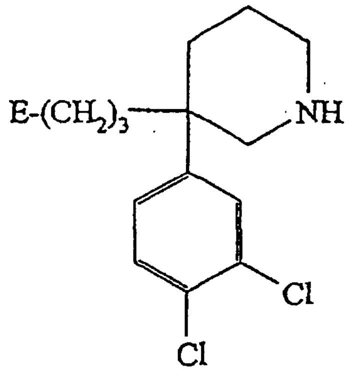

1

worin E Hydroxyl oder gegebenenfalls eine O-geschützte Gruppe darstellt, wie beispielsweise Tetrahydropyrah-2-yloxy, Benzoyloxy oder ($C_1$-$C_4$)-Alkylcarbonyloxy oder eine Gruppe

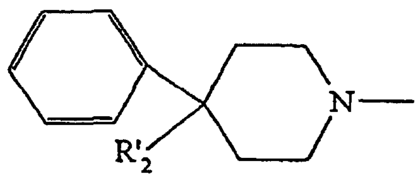

2

worin R'$_2$ wie in Anspruch 4 definiert ist,
mit Hilfe einer Schutzgruppe geschützt wird zur Herstellung einer Verbindung der Formel:

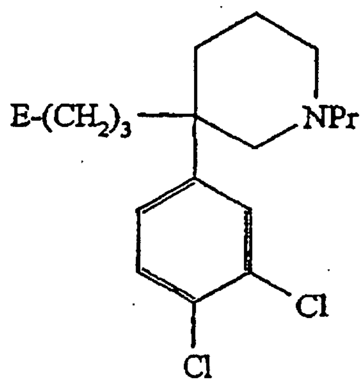

12

worin Pr eine Schutzgruppe von Stickstoff darstellt, beispielsweise tert.Butoxycarbonyl (Boc), Trityl oder Benzyl,
b1) die O-Schutzgruppe durch Einwirken einer Säure oder einer Base gegebenenfalls entfernt wird,
c1) der so erhaltene Alkohol der Formel:

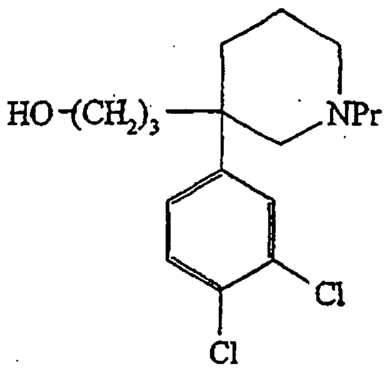

13

mit einer Verbindung der Formel

$$W\text{-}SO_2\text{-}Cl \qquad 9a$$

behandelt wird, worin W Methyl, Phenyl, Tolyl oder Trifluormethyl darstellt,
d1) das so erhaltene Sulfonat der Formel:

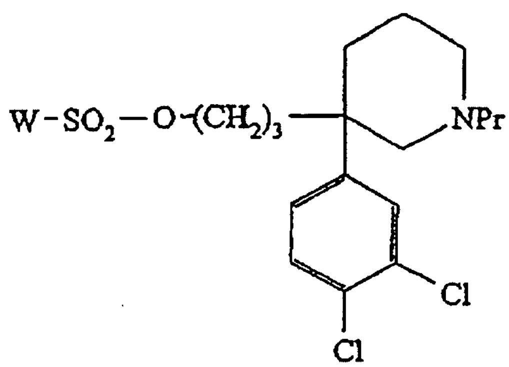

14

mit einem sekundären Amin:

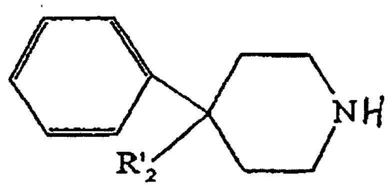

11

worin $R'_2$ wie oben definiert ist, zur Umsetzung gebracht wird zur Herstellung der Verbindung der Formel:

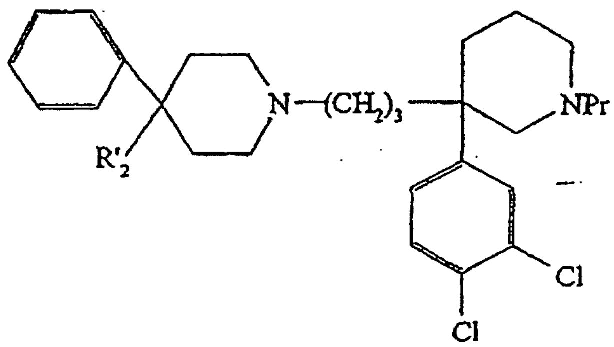

15

e1) die Entschützung des Stickstoffs in saurem Milieu vorgenommen wird,
f1) die so erhaltene Verbindung der Formel:

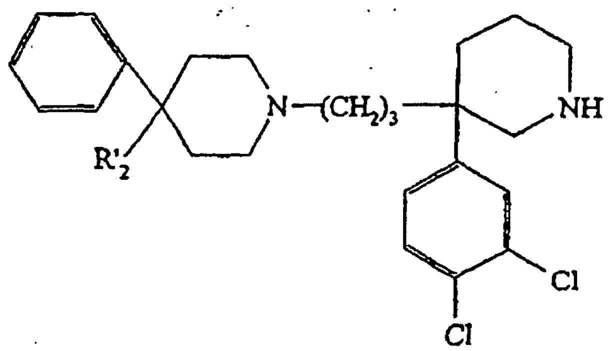

16

mit der in Anspruch 4 definierten Verbindung 3 behandelt wird,
g1) nach gegebenenfalls Entschützen des durch $R'_2$ dargestellten Hydroxyls oder Aminos das so erhaltene Produkt der Formel (I) gegebenenfalls in eines seiner Salze übergeführt wird.

**6.** Pharmazeutische Zusammensetzung, die als Wirkstoff eine Verbindung nach einem der Ansprüche 1 bis 3 enthält.

**7.** Pharmazeutische Zusammensetzung nach Anspruch 6 in Dosiseinheitsform, worin der Wirkstoff mit mindestens einem pharmazeutischen Exzipienten vermischt ist.

**8.** Pharmazeutische Zusammensetzung nach Anspruch 7, die 0,5 bis 1000 mg Wirkstoff enthält.

**9.** Pharmazeutische Zusammensetzung nach Anspruch 8, die 2,5 bis 250 mg Wirkstoff enthält.

**10.** Verwendung einer Verbindung nach einem der Ansprüche 1 bis 3 oder eines pharmazeutisch annehmbaren Salzes derselben zur Herstellung eines Medikaments zur Bekämpfung von psychischen Erkrankungen, Erkrankungen psychosomatischen Ursprungs, Hypertonie oder Krankheiten die mit Störungen der $NK_B$-abhängigen Neurotransmission oder Neuromodulation verbunden sind.

**11.** Verwendung nach Anspruch 10, worin die Verbindung 1-Benzoyl-3-(3,4-dichlorphenyl)-3-[3-(4-(acetyl-N-methylamino)-4-phenylpiperid-I-yl)propyl]piperidin-hydrochlorid in der (+)-Isomerform ist.

**Claims**

**1.** A compound which is chosen from the compounds below:

1-benzoyl-3 -(3,4-dichlorophenyl)-3 -[3 -(4-(acetyl-N-methylamino)-4-phenylpiperid-1-yl)propyl]piperidine,

1-benzoyl-3-(3,4-dichlorophenyl)-3-[3-(4-propionylamino-4-phenylpiperid-1-yl)propyl]piperidine,
1-benzoyl-3-(3,4-dichlorophenyl)-3-[3-(4-(propionyl-N-methylamino)-4-phenylpiperid-1-yl)propyl]piperidine,
1-benzoyl-3-(3,4-dichlorophenyl)-3-[3-(4-butyrylamino-4-phenylpiperid-1-yl)propyl]piperidine,
1-benzoyl-3-(3,4-dichlorophenyl)-3-[3-(4-(butyryl-N-methylamino)-4-phenylpiperid-1-yl)propyl]piperidine,
1-benzoyl-3-(3,4-dichlorophenyl)-3-[3-(4-isobutyrylamino-4-phenylpiperid-1-yl)propyl]piperidine,
1-benzoyl-3-(3,4-dichlorophenyl)-3-[3-(4-(iso-butyryl-N-methylamino)-4-phenylpiperid-1-yl)propyl]piperidine,
1-benzoyl-3-(3,4-dichlorophenyl)-3-[3-(4-valerylamino-4-phenylpiperid-1-yl)propyl]piperidine,
1-benzoyl-3-(3,4-dichlorophenyl)-3-[3-(4-(valeryl-N-methylamino)-4-phenylpiperid-1-yl)propyl]piperidine,
1-benzoyl-3-(3,4-dichlorophenyl)-3-[3-(4-isovalerylamino-4-phenylpiperid-1-yl)propyl]piperidine,
1-benzoyl-3-(3,4-dichlorophenyl)-3-[3-(4-(isovaleryl-N-methylamino)-4-phenylpiperid-1-yl)propyl]piperidine,
1-benzoyl-3 -(3,4-dichlorophenyl)-3 -[3-(4-pivaloylamino-4-phenylpiperid-1-yl)propyl]piperidine,
1-benzoyl-3-(3,4-dichlorophenyl)-3-[3-(4-(pivaloyl-N-methylamino)-4-phenylpiperid-1-yl)propyl]piperidine,

in the form of a racemate or a (+) or (-) enantiomer, and their pharmaceutically acceptable salts.

**2.** The compound of claim 1, which is 1-Benzoyl-3-(3,4-dichlorophenyl)-3-[3-(4-(acetyl-N-methylamino)-4-phenylpiperid-1-yl)propyl]piperidine in the form of the (+) isomer.

**3.** The compound of claim 1, which is 1-Benzoyl-3-(3,4-dichlorophenyl)-3-[3-(4-(acetyl-N-methylamino)-4-phenylpiperid-1-yl)propyl]piperidine hydrochloride in the form of the (+) isomer.

**4.** A process for preparing a compound of formula (I) as claimed in claim 1 and salts thereof, wherein:

a) a compound of formula:

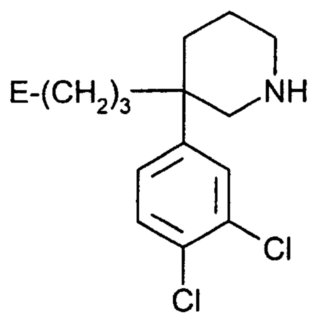

1

in which E represents a hydroxyl or optionally an O-protected group such as for example a tetrahydropyran-2-yloxy, a benzoyloxy or a $(C_1-C_4)$alkylcarbonyloxy or a group:

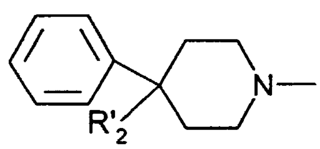

2

in which $R'_2$ represents a group acetyl-N-methylamino, propionylamino, propionyl-N-methylamino, butyrylamino, butyryl-N-methylamino, isobutyrylamino, isobutyryl-N-methylamino, valerylamino, valeryl-N-methylamino, isovalerylamino, isovaleryl-N-methylamino, pivaloylamino, pivaloyl-N-methylamino or a precursor of such a group,
is treated with a functional derivative of an acid of formula:

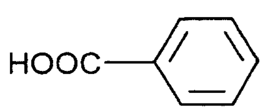

3

to obtain a compound of formula :

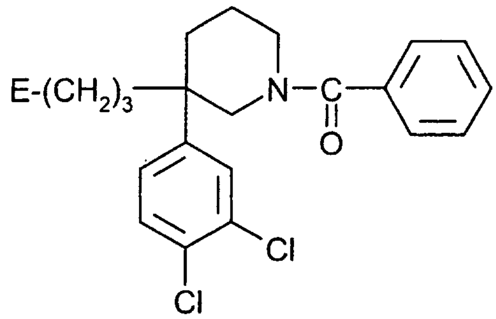

8

b) the O-protecting group is optionally removed by the action of an acid or of a base,
c) the alcohol thus obtained of formula :

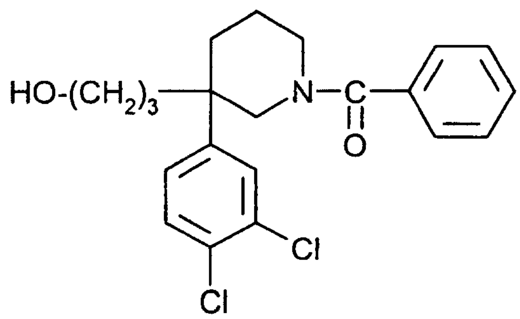

9

is treated with a compound of formula:

$W-SO_2-Cl$ 9a

in which W represents a methyl, phenyl, tolyl or trifluoromethyl group,
d) the sulfonate thus obtained of formula:

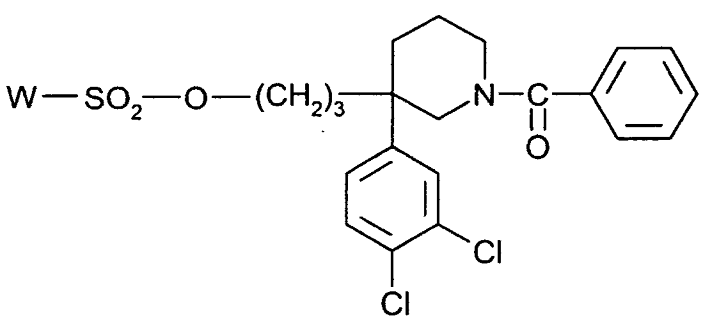

10

is reacted with a secondary amine of formula:

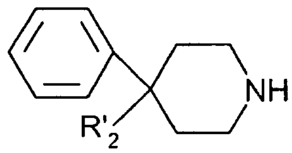

11

in which $R'_2$ is as defined above,
e) after optional deprotection of the hydroxyl or of the amino represented by $R'_2$, the product obtained is optionally converted to one of its salts.

**5.** A process for preparing a compound of formula (I) as claimed in claim 1 and salts thereof, wherein

a1) the amine function of the compound of formula:

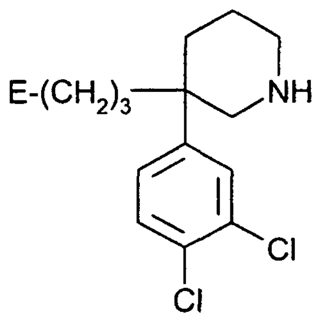

1

in which E represents a hydroxyl or optionally an O-protected group such as for example a tetrahydropyran-2-yloxy, a benzoyloxy or a $(C_1-C_4)$alkylcarbonyloxy, or a group:

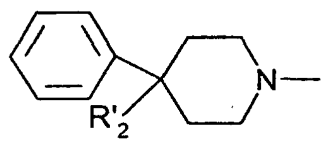

2

in which $R'_2$ is as defined in claim 4,
is protected with a protecting group in order to prepare a compound of formula:

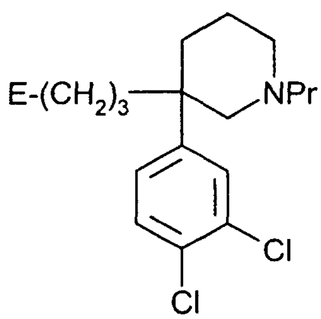

12

in which Pr represents a nitrogen-protecting group, for example a tert-butoxycarbonyl (Boc), a trityl or a benzyl,
b1) the O-protecting group is optionally removed by the action of an acid or of a base,
c1) the alcohol thus obtained of formula:

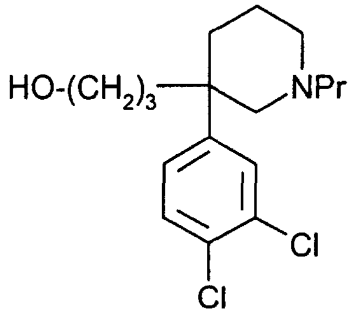

13

is treated with a compound of formula 9a :

W-$SO_2$-Cl 9a

in which W represents a methyl, phenyl, tolyl or trifluoromethyl group,
d1) the sulfonate thus obtained of formula:

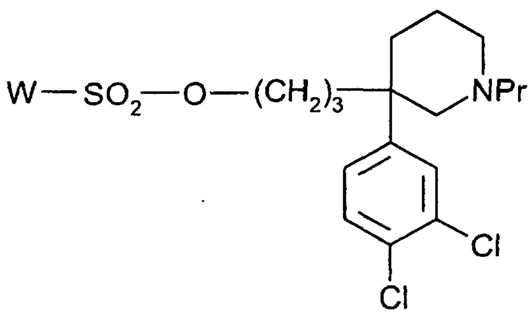

14

is reacted with a secondary amine:

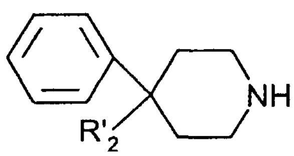

11

to give the compound of formula:

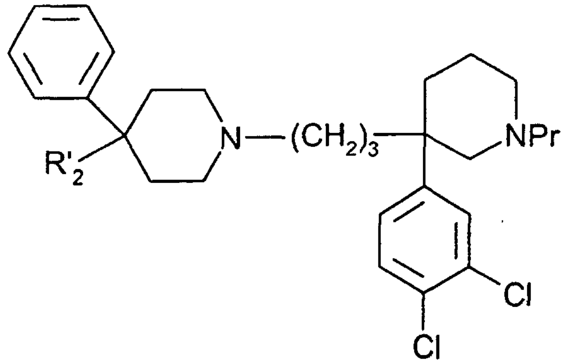

15

e1) the nitrogen is deprotected in acidic medium,
f1) the compound thus obtained of formula:

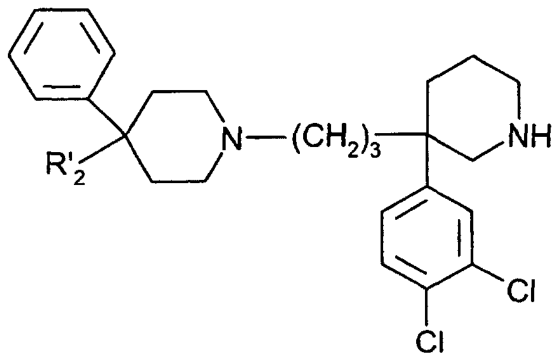

16

is treated with compound 3 defined in claim 4,
g1) after optional deprotection of the hydroxyl or of the amino represented by $R'_2$, the product of formula (I) obtained is optionally converted to one of its salts.

**6.** A pharmaceutical composition containing, as active principle, a compound of one of claims 1 to 3.

**7.** The pharmaceutical composition of claim 6, under the form of dosage unit, in which the active principle is mixed with at least one pharmaceutically excipient.

**8.** The pharmaceutical composition of claim 7, containing from 0.5 to 1000 mg of active principle.

**9.** The pharmaceutical composition of claim 8, containing from 2.5 to 250 mg of active principle.

**10.** Use of a compound of one of claims 1 to 3, or a pharmaceutically acceptable salt thereof, for the preparation of a medicament intended for the treatment of psychiatric diseases, diseases of psychosomatic origin, hypertension, or pathologies linked to $NK_B$-dependent neurotransmission or neuromodulation disorders.

**11.** The use of claim 10, wherein said compound is 1-Benzoyl-3-(3,4-dichlorophenyl)-3-[3-(4-(acetyl-N-methylamino)-4-phenylpiperid-1-yl)propyl]piperidine hydrochloride in the form of the (+) isomer.